(19) Europäisches Patentamt
European Patent Office
Office européen des brevets

(11) EP 0 741 133 B1

(12) **EUROPÄISCHE PATENTSCHRIFT**

(45) Veröffentlichungstag und Bekanntmachung des Hinweises auf die Patenterteilung:
**28.01.2004 Patentblatt 2004/05**

(21) Anmeldenummer: **96106423.5**

(22) Anmeldetag: **24.04.1996**

(51) Int Cl.[7]: **C07D 263/20**, C07D 401/12, C07D 401/04, C07D 413/12, C07D 413/06, C07D 211/58, C07D 211/22, C07D 211/44, C07D 207/26, C07D 243/24, C07C 257/18, A61K 31/155, A61K 31/42, A61K 31/445, A61K 31/495, A61K 31/55, A61K 31/34, A61K 31/38

(54) **Adhäsionsrezeptor-Antagonisten**

Adhesion receptor antagonists

Antagonistes des récepteurs d'adhésion

(84) Benannte Vertragsstaaten:
**AT BE CH DE DK ES FI FR GB GR IE IT LI LU NL PT SE**
Benannte Erstreckungsstaaten:
**LT LV SI**

(30) Priorität: **05.05.1995 DE 19516483**

(43) Veröffentlichungstag der Anmeldung:
**06.11.1996 Patentblatt 1996/45**

(73) Patentinhaber: **MERCK PATENT GmbH**
**64293 Darmstadt (DE)**

(72) Erfinder:
- **Gante, Joachim, Prof.**
  **64289 Darmstadt (DE)**
- **Juraszyk, Horst, Dr.**
  **64342 Seeheim (DE)**
- **Raddatz, Peter, Dr.**
  **64342 Seeheim (DE)**
- **Wurziger, Hanns, Dr.**
  **64291 Darmstadt (DE)**
- **Bernotat-Danielowski, Sabine, Dr.**
  **61231 Bad Nauheim (DE)**
- **Melzer, Guido, Dr.**
  **65719 Hofheim/Ts. (DE)**

(56) Entgegenhaltungen:

| | |
|---|---|
| **EP-A- 0 381 033** | **EP-A- 0 459 256** |
| **EP-A- 0 462 960** | **EP-A- 0 542 363** |
| **EP-A- 0 623 615** | **EP-A- 0 645 376** |
| **EP-A- 0 697 408** | **EP-A- 0 710 657** |
| **WO-A-93/00095** | **WO-A-94/14776** |
| **WO-A-95/18111** | **WO-A-95/18619** |
| **DE-A- 4 234 295** | **US-A- 5 344 957** |

- **BIOORGANIC & MEDICINAL CHEMISTRY, Bd. 2, Nr. 9, 1994, Seiten 897-908, XP000196285 W.E. BONDINELL ET AL.: "Design of a Potent and Orally Active Nonpeptide Platelet Fibrinogen Receptor (GPIIb/IIIa) Antagonist"**
- **BIOORGANIC & MEDICINAL CHEMISTRY, Bd. 2, Nr. 9, 1994, Seiten 881-95, XP000575048 P.R. BOVY ET AL.: "Design of Orally Active, Non-Peptide Fibrinogen Receptor Antagonists. An Evolutionary Process from the RGD Sequence to Novel Anti-Platelet Aggregation Agents"**
- **CIRCULATION, Bd. 91, Nr. 2, 1995, Seiten 403-10, XP000196284 N.S. NICHOLSON ET AL.: "SC-54684A: An Orally Active Inhibitor of Platelet Aggregation"**
- **JOURNAL OF MEDICINAL CHEMISTRY, Bd. 38, Nr. 13, 1995, Seiten 2378-94, XP000579664 J.A. ZABLOCKI ET AL.: "Potent in Vitro and in Vivo Inhibitors of Platelet Aggregation Based Upon the Arg-Gly-Asp Sequence of Fibrinogen. (Aminobenz- amidino)succinyl (ABAS) Series of Orally Active Fibrinogen Receptor Antagonists"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**(Forts. nächste Seite)**

- **JOURNAL OF MEDICINAL CHEMISTRY, Bd. 37, Nr. 23, 1994, Seiten 3882-5, XP000579663 C.D. ELDRED ET AL.: "Orally Active Non-Peptide Fibrinogen Receptor (GpIIb/IIIa) Antagonists: Identification of 4-[4-[4-(Aminoiminomethyl)phenyl]-1-piperazinyl]-1-piperidineacetic Acid as a Long-Acting, Broad Spectrum Antithrombotic Agent"**
- **THROMBOSIS AND HAEMOSTASIS, Bd. 70, Nr. 5, 1993, Seiten 817-21, XP000196280 J.-P. CARTEAUX ET AL.: "Ro 44-9883, a New Non-Peptidic GPIIb-GPIIIa Antagonist Prevents Platelet Loss in a Guinea Pig Model of Extracorporeal Circulation"**
- **JOURNAL OF MEDICINAL CHEMISTRY, Bd. 35, Nr. 23, 1992, Seiten 4393-407, XP000561169 L. ALIG ET AL.: "Low Molecular Weight, Non-Peptide Fibrinogen Receptor Antagonists"**
- **JOURNAL OF THE AMERICAN CHEMICAL SOCIETY, Bd. 115, Nr. 19, 1993, Seiten 8861-2, XP000196275 T.W. KU ET AL.: "Direct Design of a Potent Non-Peptide Fibrinogen Receptor Antagonist Based on the Structure and Conformation of a Highly Constrained Cyclic RGD Peptide"**
- **JOURNAL OF MEDICINAL CHEMISTRY, Bd. 38, Nr. 1, 1995, Seiten 9-12, XP000579662 T.W. KU ET AL.: "Potent Non-peptide Fibrinogen Receptor antagonists Which Present an Alternative Pharmacophore"**


Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft neue Adhäsionsrezeptor-Antagonisten der Formel I

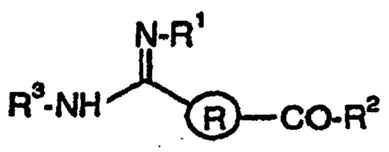

I,

worin

R 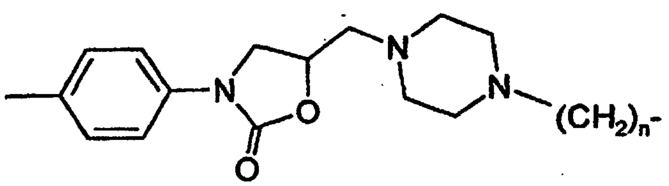

,

mit n = 1 oder 2,

$R^1$ H,

$R^2$ OA oder OAr,

$R^3$ A-CO, Ar-CO, Het-CO, Het-O-CO, Ar-O-CO, A-O-CO, Ar-$SO_2$ oder A-$SO_2$;

A Alkyl mit 1 bis 6 C-Atomen;

Ar unsubstituiertes oder ein-, zwei- oder dreifach durch A, F, Cl, Br, I, OA, -O-$CH_2$-O-, COOA, COOH, $CF_3$, OH, $NO_2$, CN, O-CO-A, $NH_2$, NHA oder $NA_2$ substituiertes Aryl mit 6 bis 10 C-Atomen, Diphenylmethyl oder Benzyl und

Het einen ein- oder zweikernigen gesättigten, ungesättigten oder aromatischen Heterozyklus mit 1 bis 4 N-, O- und/oder S-Atomen, der unsubstituiert oder einfach durch F, Cl, Br, $CF_3$, A, OH, OA, CN oder $NO_2$ substituiert sein kann,

bedeuten,
sowie deren physiologisch unbedenkliche Salze.

Ähnliche Verbindungen sind aus der EP-A1-0 623 615 (DE 43 14 378) bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden; daß die Verbindungen der Formel I sowie ihre Solvate und Salze bei guter Verträglichkeit wertvolle pharmakologische Eigenschaften besitzen. Vor allem wirken sie als Integrin-Inhibitoren, wobei sie insbesondere die Wechselwirkungen der $\beta_3$- oder $\beta_5$-Integrin-Rezeptoren mit Liganden hemmen. Besondere Wirksamkeit zeigen die Verbindungen im Falle der Integrine $a_v\beta_3$, $a_v\beta_5$ und $a_{IIb}\beta_3$. Diese Wirkung kann z.B. nach der Methode nachgewiesen werden, die von J.W. Smith et al. in J. Biol. Chem. 265, 12267-12271 (1990) beschrieben wird. Insbesondere hemmen sie die Bindung von Fibrinogen, Fibronectin und des von-Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Angina pectoris, osteolytische Erkrankungen, insbesondere Osteoporose und Restenose nach Angioplastie, Ischämien, Entzündungen, Arteriosklerose und von akutem Nierenversagen verwendet werden. Die Verbindungen hemmen oder verhindern die Gefäßausbildung und zeigen somit einen antiangiogenetischen Effekt. Ferner haben die Verbindungen einen Effekt auf Tumorzellen, indem sie deren Metastasierung hemmen. Somit können sie auch als Anti-Tumor-Mittel eingesetzt werden.

Es gibt Hinweise, daß Tumorzellen durch Mikrothrombi in die Gefäße gelangen und somit vor der Detektion durch die Zellen des Immunsystems geschützt sind. Ebenso wirken Mikrothrombi unterstützend auf die Bindung der Tumorzellen an die Gefäßwände. Da die Bildung der Mikrothrombi im Zusammenhang mit der Fibrinogen-Bindung zum Fibrinogen-Rezeptor (Glycoprotein IIb/IIIa) steht, gelten Fibrinogen-Bindungs-Inhibitoren ebenfalls als Metastase-Inhibitoren. Sie verhindern durch ihre antiangiogenetischen Fähigkeiten die Blut- und Nährstoffversorgung der Tumorzellen.

Die Verbindungen eignen sich zudem als antimikrobielle Wirkstoffe, die Infektionen, wie sie beispielsweise durch Bakterien, Pilze oder Hefen ausgelöst werden, verhindern können. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika. Antimikrobielle Aktivitäten der Verbindungen können z.B. nach der Methode von P. Valentin-Weigand et al., beschrieben in Infection and Immunity, 2851-2855 (1988), nachgewiesen werden.

Die anderen Eigenschaften der Verbindungen können nach Methoden nachgewiesen werden, die in der EP-A1-0 462 960 beschrieben sind. Die Hemmung der Fibrinbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, (1962)) nachweisen.

Verbindungen der angegebenen Formel I sowie deren Salze können durch ein Verfahren hergestellt werden, dadurch gekennzeichnet, daß man

(i) eine Verbindung der Formel I aus einem ihrer funktionellen Derivate durch Behandeln mit einem solvolysierenden oder hydrogenolysierenden Mittel in Freiheit setzt, oder daß man

(ii) eine Verbindung der Formel II

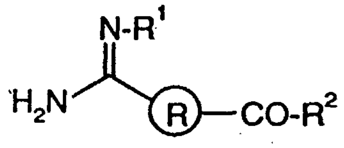

II,

worin

R, $R^1$ und $R^2$ die angegebenen Bedeutungen besitzen,

mit einer Verbindung der Formel III

$$R^3\text{-}X \qquad \text{III,}$$

worin

$R^3$ die angegebene Bedeutung besitzt und

X OH, F, Cl, Br, I oder eine andere leicht verdrängbare Abgangsgruppe ist, bedeutet,

umsetzt, oder daß man

(iii) zur Herstellung einer Verbindung der Formel I nach Anspruch 1

eine Verbindung der Formel IV

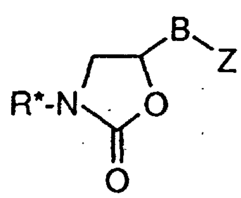

IV,

worin

R*

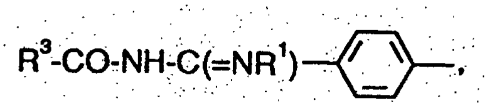

wobei $R^1$ und $R^3$, die in Anspruch 1 angegebenen Bedeutungen besitzen, B die Gruppe $CH_2$ bedeutet und

Z Cl, Br, I, OH oder eine reaktionsfähig veresterte OH-Gruppe bedeutet,

mit einer Verbindung der Formel Va

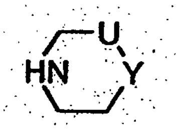 Va,

worin

Y $-N-(CH_2)_n-COR^2$, wobei $R^2$ und n die in Anspruch 1 angegebenen Bedeutungen besitzen und U gleich $CH_2$ ist,

oder daß man eine Verbindung der Formel VI

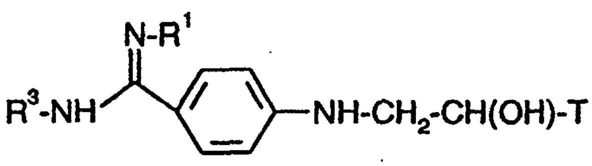 VI,

worin

T

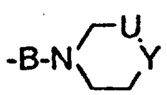

wobei B, L, U und Y sowie

$R^1$ und $R^3$ die bereits angegebenen Bedeutungen haben, mit einem reaktionsfähigen Derivat der Kohlensäure umsetzt, oder daß man

zur Herstellung einer Verbindung der Formel nach Anspruch 1, in einer Verbindung, die an sich der Formel I entspricht

(iii) einen Rest $R^1$ in einen anderen Rest $R^1$ umwandelt, indem man

- alkyliert oder acyliert, oder daß man

(iv) einen Rest $R^2$ in einen anderen Rest $R^2$ umwandelt, indem man

- ein Amid alkyliert
- eine Cyan-Gruppe vollständig oder teilweise hydrolisiert
- eine COOH-Gruppe verestert oder
- eine COOH- oder COOA-Gruppe in ein Amid überführt, oder daß man,

(v) eine Verbindung der Formel I nach Anspruch 1 durch Behandeln mit einer Säure oder Base in eines ihrer Salze überführt.

Die Verbindungen der Formel I besitzen mindestens ein chirales Zentrum und können daher in mehreren enantiomeren Formen auftreten. Alle diese Formen (z.B. R- und S-Formen) und deren Gemische (z.B. die RS-Formen) sind in der Formel I eingeschlossen.

Vor- und nachstehend haben sämtliche Reste bzw. Parameter, die bei den Formeln I bis X angegebenen Bedeutungen, falls nicht ausdrücklich etwas anderes angegeben ist. Falls mehrere gleich bezeichnete Gruppen oder Parameter im Molekül vorhanden sind, können sie unabhängig voneinander verschiedene Definitionen annehmen.

In den vorstehenden Formeln hat die Gruppe A 1-6, vorzugsweise 1, 2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, hexyl, 1-, 2-, 3- oder 4-Methylpentyl.

Der Rest ® steht für

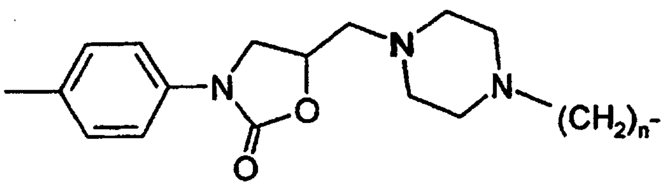

mit n = 1 oder 2,

$R^1$ ist Wasserstoff.

$R^2$ ist OA oder OAr, während $R^3$ vorzugsweise A-CO, Ar-CO, Het-CO, Ar-O-CO, Ar-$SO_2$ oder A-$SO_2$ ist.

Ar ist vorzugsweise Phenyl, Benzyl oder Diphenylmethyl, ferner aber auch bevorzugt 1- oder 2-Naphthyl, wobei die genannten Reste vorzugsweise unsubstituiert sind, aber auch ein-, zwei- oder dreifach durch die genannten Reste, insbesondere A, F, Cl, Br, Methylendioxy, COOH, $COOCH_3$, O-CO-A, $COOC_2H_5$, $CF_3$, OH oder OA, substituiert sein können.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- öder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3-oder 4-4H-Thiopyranyl, 3- oder 4-Pyridazinyl, Pyrazinyl, 2-, 3-, 4-, 5-, 6-oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6- 7- oder 8-Chinazolinyl. Die heterocyclischen Reste können auch teilweise oder vollständig hydriert sein.

Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4-oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, - 3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder - 6-pyridyl, 1,2,3,6-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolinyl, 1,2,3,4-Tetrahydro-1-, -2-, -3-, -4-, -5-, -6-, -7- oder 8-isochinolinyl.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Bei

einigen bevorzugten Gruppen von Verbindungen die der Formel I entsprechen, bedeutet

$R^3$ Benzoyl, 1- oder 2-Naphthyl, Furoyl, Thienoyl oder Carbobenzoxy und $R^2$ OA.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner J. Med. Chem. 37, 3881-3886 (1994), EP-A1-0 381 033,
EP-A1-0 462 960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, insbesondere solche, die an Stelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch an Stelle einer Gruppe -COOH eine Gruppe -COOR" tragen, worin R" eine Hydroxyschutzgruppe bedeutet.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfembar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen.

Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfembar sind, nachdem die gewünschte chemische Reaktion an einer anderen Stelle des Moleküls durchgeführt worden ist. Typisch für solche Gruppen sind die obengenannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe an Stelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure,

Ether wie Tetrahydrofuran oder Dioxan, Amide wie Dimethylformamid (DMF), halogenierte Kohlenwasserstoffe wie Dichlormethan, Sulfoxide wie Dimethylsulfoxid (DMSO), ferner auch Alkohole wie Methanol, Ethanol oder Isopropanol sowie Wasser. Ferner kommen Gemische der vorgenannten Lösungsmittel in Frage.

Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten werden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können bevorzugt auch durch Reaktion einer Verbindung der Formel II mit einem Carbonsäurederivat der Formel III erhalten werden. Dabei bedient man sich zweckmäßig der an sich bekannten Methoden zur Acylierung von Aminen.

Die Gruppe X in der Formel III bedeutet vorzugsweise Cl, Br, I, $C_1$-$C_6$-Alkylsulfonyloxy wie Methan- oder Ethansulfonyloxy oder $C_6$-$C_{10}$-Arylsulfonyloxy wie Benzol-, p-Toluol- oder 1- oder 2-Naphthalinsulfonyloxy.

Die Reaktion gelingt vorzugsweise in Gegenwart einer zusätzlichen Base, z.B. eines Alkali- oder Erdalkalimetall-hydroxids oder carbonats wie Natrium-, Kalium- oder Calciumhydroxid, Natrium-, Kalium- oder Calciumcarbonat, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Falls die Fluchtgruppe von Iod verschieden ist, empfiehlt sich ein Zusatz eines Iodids wie Kaliumiodid.

Die Ausgangsstoffe der Formel II sind in der Regel bekannt und können z.B. nach den in EP 0 623 615 (korrespondierend zu DE 43 14 378) beschriebenen Methoden hergestellt werden.

Zur Herstellung eines Amidins der Formel II kann man an ein Nitril der Formel II Ammoniak anlagern. Die Anlagerung erfolgt bevorzugt mehrstufig, indem man in an sich bekannter Weise a) das Nitril mit $H_2S$ in ein Thioamid umwandelt, das mit einem Alkylierungsmittel, z.B. $CH_3I$, in den entsprechenden S-Alkyl-imidothioester übergeführt wird, welcher seinerseits mit $NH_3$ zum Amidin reagiert, b) das Nitril mit einem Alkohol, z.B. Ethanol in Gegenwart von HCl in den entsprechenden Imidoester umwandelt und diesen mit Ammoniak behandelt, oder c) das Nitril mit Lithium-bis-(trimethylsilyl)-amid umsetzt und das Produkt anschließend hydrolysiert.

Analog sind die entsprechenden N-Hydroxy-amidine der Formel II aus den Nitrilen erhältlich, wenn man nach a) oder b), aber mit Hydroxylamin an Stelle von Ammoniak arbeitet. Diese Produkte können dann auch modifiziert werden, indem man sie z.B. mit Wasserstoffgas reduziert.

Die Verbindungen der Formel III sind bekannt und zum größten Teil kommerziell erhältlich.

Die Umsetzung der Verbindungen II mit den Verbindungen III erfolgt wie bereits zuvor beschrieben.

Ferner ist es möglich, eine Verbindung der Formel I zu erhalten, indem man eine Verbindung der Formel IV mit einer Verbindung der Formel Va umsetzt.

Die Verbindungen der Formel IV sind teilweise aus EP 0 623 615 bekannt oder können nach den dort beschriebenen Methoden hergestellt werden.

Sie können z.B. hergestellt werden durch Reaktion eines substituierten Anilins der Formel $R^*$-$NH_2$ mit einer Verbindung der Formel $R^5CH_2$-$CHR^6$-$CH_2OH$ (worin $R^5$ Cl, Br oder eine andere geeignete Abgangsgruppe bedeutet und $R^6$OH oder $R^5$ und $R^6$ zusammen auch O sind) zu einer Verbindung der Formel $R^*$-NH-$CH_2$-$CHR^8$-$CH_2OH$ (worin $R^8$ OH bedeutet), Reaktion mit einem Derivat der Kohlensäure wie Diethylcarbonat zu 3-R*-5-hydroxymethyl-2-oxazolidinonen und gegebenenfalls Umwandlung der Hydroxymethylgruppe in eine $CH_2Z'$-Gruppe (wobei Z eine Fluchtgruppe bedeutet), z.B. mit $SOCl_2$, $SOBr_2$, Methansulfonylchlorid oder p-Toluolsulfonylchlorid. Die Verbindungen der Formel Vb sind in der Regel bekannt oder in Analogie zu bekannten Verbindungen aus geeigneten Phenolderivaten oder aus Phenol herstellbar. Das gleiche gilt für die Verbindungen der Formel Va. Sie können nach an sich bekannten Methoden aus Piperidin- oder Piperazinderivaten hergestellt werden.

Die Umsetzung gelingt unter ähnlichen Bedingungen wie sie zuvor für die Reaktion zwischen Verbindungen II und III beschrieben wurde.

Verbindungen der Formel I können ferner erhalten werden durch Reaktion einer Verbindung der Formel IV (oder eines reaktionsfähigen Derivats davon) mit einem reaktiven Derivat der Kohlensäure.

Als Kohlensäurederivate eignen sich insbesondere Dialkylcarbonate wie Diethylcarbonat, ferner auch Chlorameisensäurealkylester wie Ethyl-chlorformiat. Bevorzugt dient das Kohlensäurederivat, das zweckmäßig im Überschuß eingesetzt wird, auch als Lösungs- bzw. Suspensionsmittel.

Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kaliumtert.-butylat. Man arbeitet zweckmäßig bei Reaktionstemperaturen zwischen 0 und 150°, vorzugsweise zwischen 70 und 120°.

Die Ausgangsstoffe der Formel IV sind in der Regel neu. Sie sind z.B. erhältlich durch Funktionalisierung der oben genannten Verbindungen der Formel R*-NH-$CH_2$-CH(OH)-$CH_2$OH zu Verbindungen der Formel R*-NH-$CH_2$-CH (OH)-$CH_2$-Z und Reaktion mit Verbindungen der Formel Va.

Als inerte Lösungsmittel eignen sich insbesondere Alkohole wie Methanol, Ethanol oder Isopropanol, n-Butanol oder tert.-Butanol; Ether wie Diethylether, Diisopropylether, THF oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Bevorzugte Reaktionstemperaturen liegen zwischen Raumtemperatur und dem Siedepunkt des gewählten Lösungsmittels.

Weiterhin ist es möglich, in einer Verbindung der Formel I einen Rest $R^2$ in einen anderen Rest $R^2$ umzuwandeln, indem man einen Ester der Formel I verseift oder eine Carbonsäure der Formel I verestert.

Zur Veresterung kann man eine Säure der Formel I ($R^2$ = H) mit einem Überschuß eines Alkohols der Formel $R^2$-OH ($R^2$ = A oder Benzyl) behandeln, zweckmäßig in Gegenwart einer starken Säure wie Salzsäure oder Schwefelsäure bei Temperaturen zwischen 0 und 100, vorzugsweise 20 und 50°.

Umgekehrt kann ein Ester der Formel I (R2 = A oder Benzyl), in die entsprechende Säure der Formel I ($R^2$ = H) umgewandelt werden, zweckmäßig durch Solvolyse oder Hydrogenolyse nach einer der oben angegebenen Methoden, z.B. mit NaOH oder KOH in Wasser-Dioxan bei Temperaturen zwischen 0 und 40°, vorzugsweise 10 und 30°.

Ebenso kann man Cyangruppen vollständig oder teilweise hydrolisieren.

Ferner ist es möglich, daß man einen Rest $R^1$ und/oder $R^3$ in einen anderen Rest $R^1$ und/oder $R^3$ umwandelt.

Insbesondere kann man primäre oder sekundäre Aminogruppen alkylieren, acylieren, amidinieren oder mit konventionellen Aminoschutzgruppen oder Alkyl- oder Arylsulfonylgruppen versehen bzw. in umgekehrter Weise durch Entfernung dieser Gruppen freisetzen.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Malein-säure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel I ($R^2$ = H) durch Umsetzung mit entsprechenden Basen in ihre Metall- oder Ammoniumsalze umzuwandeln, z.B. ihre Natrium-, Kalium- oder Calciumsalze.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenyl-glycin) gefüllten Säule; als Laufmittel eignet sich z.B. ein Gemisch Hexan/Isopropanol/ Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Die Verbindungen der Formel I können ebenso in tautomeren Formen auftreten. Alle diese Tautomeren werden von der Erfindung eingeschlossen.

Die neuen Verbindungen der Formel I und ihre physiologisch unbedenklichen Salze können zur Herstellung pharmazeutischer Präparate verwendet werden, indem man sie zusammen mit mindestens einem Trägeroder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459 256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, filtriert über eine Ionenaustauschersäule, trocknet die organische Phase über Natriumsulfat, dampft ein, lyophilisiert gegebenenfalls und reinigt durch Chromatographie an Kieselgel und/oder Kristallisation. In den nachfolgenden Beispielen bedeutet "4-piperidylethyl" stets "2-(4-piperidyl)-ethyl", "4-piperidylpropyl" stets "3-(4-piperidyl)-propyl" und "4-piperidylbutyl" stets "4-(4-piperidyl)-butyl". Ebenso bedeutet "4-piperazinylethyl" stets "2-(4-piperazinyl)-ethyl", "4-piperazinylpropyl" "3-(4-piperazinyl)-propyl" und "4-piperazinylbutyl" "4-(4-piperazinyl)-butyl". Eingeschlossen sind hierbei auch die mit Schutzgruppen versehenen Derivate, z.B. die BOC-geschützten Verbindungen.

**Beispiel 1**

Zu einer Lösung von 1,2 g 4-Ethoxycarbonylmethyl-piperazin ("A") in 20 ml DMF gibt man 3,0 g 3-[4-(N-Benzoylamidino)-phenyl]-5-methansulfonyloxy-methyl-oxazolidin-2-on [erhältlich durch Reaktion von 4-(5-Oxo-1,2,4-oxadiazolin-3-yl)-anilin mit 2,3-Epoxypropan-1-ol zu N-[4-(5-Oxo-1,2,4-oxadia-zolin-3-yl)-phenyl]-2,3-dihydroxy-propyl-amin, Umsetzung mit Diethylcarbonat in Gegenwart von K-tert.-butylat zu 3-[4-(5-Oxo-1,2,4-oxadiazolin-3-yl)-phenyl]-5-hydroxymethyl-oxazolidin-2-on, reduktive Spaltung der 5-Oxo-1,2,4-oxadiazolin-Gruppe, Umsetzung mit Benzoylchlorid und anschließende Veresterung mit Methansulfonylchlorid], gelöst in 10 ml DMF, hinzu und rührt 60 Min. bei Raumtempe-ratur. Nach Entfernung des Lösungsmittels und üblicher Aufarbeitung erhält man das 3-[4-(N-Benzoyl-amidino)-phenyl]-5-(4-ethoxycarbonylmethyl-piperazinomethyl)-oxazolidin-2-on, F. 114°.

Analog erhält man durch Umsetzung von "A"

mit 3-[4-(N-Benzoylamidino)-phenyl]-5(R)-methansulfonyloxy-methyl-oxazolidin-2-on
3-[4-(N-Benzoylamidino)-phenyl]-5(R)-(4-ethoxycarbonylmethyl-piperazinomethyl)-oxazolidin-2-on, F. 150°, $[\alpha]_{20}^{D}$ = +33,4° (DMSO);

mit 3-[4-(N-Benzoylamidino)-phenyl]-5(S)-methansulfonyloxy-methyl-oxazolidin-2-on
3-[4-(N-Benzoylamidino)-phenyl]-5(S)-(4-ethoxycarbonylmethyl- piperazinomethyl)-oxazolidin-2-on, F. 149°, $[\alpha]_{20}^{D}$ = -32,6° (DMSO);

mit 3-[4-(N-Benzyloxycarbonylamidino)-phenyl]-5-methansulfonyloxymethyl-oxazolidin-2-on das
3-[4-(N-Benzyloxycarbonylamidino)-phenyl]-5-(4-ethoxycarbonylmethyl-piperazinomethyl)-oxazolidin-2-on, F. 129°;

mit 3-[4-(N-Phenoxycarbonylamidino)-phenyl]-5-methansulfonyloxymethyl-oxazolidin-2-on das
3-[4-(N-Phenoxycarbonylamidino)-phenyl]-5-(4-ethoxycarbonylmethyl-piperazinomethyl)-oxazolidin-2-on, F. 176°;

mit 3-[4-(N-(3-Pyridylcarbonyl)-amidino)-phenyl]-5-methansulfonyloxy-methyl-oxazolidin-2-on das
3-[4-(N-(3-Pyridylcarbonyl)-amidino)-phenyl]-5-(4-ethoxycarbonylmethyl-piperazinomethyl)-oxazolidin-2-on, F. 134-135°;

mit 3-[4-(N-(1-Methyl-piperidyl-4-oxycarbonyl)-amidino)-phenyl]-5-methansulfonyloxy-methyl-oxazolidin-2-on das
3-[4-(N-(1-Methyl-piperidyl-4-oxycarbonyl)-amidino)-phenyl]-5-(4-ethoxycarbonylmethyl-piperazinomethyl)-oxazolidin-2-on, Dihydrochlorid, F. 91-93°;

mit 3-[4-(N-1-Naphthoylamidino)-phenyl]-5-methansulfonyloxy-methyl- oxazolidin-2-on das
3-[4-(N-1-Naphthoylamidino)-phenyl]-5-(4-ethoxycarbonylmethyl-piperazinomethyl)-oxazolidin-2-on, F. 160-161°;

mit 3-[4-(N-2-Naphthoylamidino)-phenyl]-5-methansulfonyloxy-methyl-oxazolidin-2-on das
3-[4-(N-2-Naphthoylamidino)-phenyl]-5-(4-ethoxycarbonylmethyl- piperazinomethyl)-oxazolidin-2-on;

mit 3-[4-(N-2-Furoylamidino)-phenyl]-5-methansulfonyloxy-methyl-oxazolidin-2-on das
3-[4-(N-2-Furoylamidino)-phenyl]-5-(4-ethoxycarbonylmethyl- piperazinomethyl)-oxazolidin-2-on, F. 172-173°;

mit 3-[4-(N-3-Furoylamidino)-phenyl]-5-methansulfonyloxy-methyl- oxazolidin-2-on das
3-[4-(N-3-Furoylamidino)-phenyl]-5-(4-ethoxycarbonylmethyl- piperazinomethyl)-oxazolidin-2-on;

mit 3-[4-(N-2-Thienoylamidino)-phenyl]-5-methansulfonyloxy-methyl- oxazolidin-2-on das
3-[4-(N-2-Thienoylamidino)-phenyl]-5-(4-ethoxycarbonylmethyl- piperazinomethyl)-oxazolidin-2-on;

mit 3-[4-(N-3-Thienoylamidino)-phenyl]-5-methansulfonyloxy-methyl- oxazolidin-2-on das
3-[4-(N-3-Thienoylamidino)-phenyl]-5-(4-ethoxycarbonylmethyl- piperazinomethyl)-oxazolidin-2-on;

mit 3-[4-(N-Methoxycarbonyl-amidino)-phenyl]-5-methansulfonyloxymethyl-oxazolidin-2-on das
3-[4-(N-Methoxycarbonyl-amidino)-phenyl]-5-(4-ethoxycarbonylmethyl-piperazinomethyl)-oxazolidin-2-on, 186-187°.

**Beispiel 2**

Analog Beispiel 1 erhält man durch Umsetzung von 1,2 g 4-Ethoxycarbonylethyl-piperazin ("B") in 20 ml DMF mit 3,0 g 3-[4-(N-Benzoylamidino)-phenyl]-5-methansulfonyloxy-methyl-oxazolidin-2-on [erhältlich gemäß Beispiel 1], gelöst in 10 ml DMF, nach Entfernung des Lösungsmittels und üblicher Aufarbeitung das 3-[4-(N-Benzoylamidino)-phenyl]-5-(4-ethoxycarbonylethyl-piperazinomethyl)-oxazolidin-2-on, F. 163-164°.
Analog erhält man durch Umsetzung von "B"

mit 3-[4-(N-Benzoylamidino)-phenyl]-5(S)-methansulfonyloxy-methyl oxazolidin-2-on
3-[4-(N-Benzoylamidino)-phenyl]-5(S)-(4-ethoxycarbonylethyl- piperazinomethyl)-oxazolidin-2-on, F. 149-150°, $[\alpha]_{20}^{D}$ = -32,6° (DMSO);

mit 3-[4-(N-Benzoylamidino)-phenyl]-5(R)-methansulfonyloxy-methyl-oxazolidin-2-on
3-[4-(N-Benzoylamidino)-phenyl]-5(R)-(4-ethoxycarbonylethyl- piperazinomethyl)-oxazolidin-2-on, F. 225-226°, $[\alpha]_{20}^{D}$ = +33,0° (DMSO);

mit 3-[4-(N-Benzyloxycarbonylamidino)-phenyl]-5-methansulfonyloxymethyl-oxazolidin-2-on das
3-[4-(N-Benzyloxycarbonylamidino)-phenyl]-5-(4-ethoxycarbonylethyl-piperazinomethyl)-oxazolidin-2-on, F. 130-131°;

mit 3-[4-(N-Benzyloxycarbonylamidino)-phenyl]-5R-methansulfonyloxymethyl-oxazolidin-2-on das 3-[4-(N-Benzyloxycarbonylamidino)-phenyl]-5R-(4-ethoxycarbonylethyl-piperazinomethyl)-oxazolidin-2-on, F. 133-134°, $[\alpha]_{20}^{D}$ = +29,5° (DMSO);

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-ethoxycarbonylethyl- piperazinomethyl)-oxazolidin-2-on;

mit 3-[4-(N-(3-Pyridylcarbonyl)-amidino)-phenyl]-5-methansulfonyloxy methyl-oxazolidin-2-on das 3-[4-(N-(3-Pyridylcarbonyl)-amidino)-phenyl]-5-(4-ethoxycarbonylethyl-piperazinomethyl)-oxazolidin-2-on;

mit 3-[4-(N-Methoxycarbonyl-amidino)-phenyl]-5-methansulfonyloxymethyl-oxazolidin-2-on das 3-[4-(N-Methoxycarbonyl-amidino)-phenyl]-5-(4-ethoxycarbonylethyl- piperazinomethyl)-oxazolidin-2-on, F. 168°;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4- ethoxycarbonylethyl-piperazinomethyl)-oxazolidin-2-on;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4- ethoxycarbonylethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-ethoxycarbonylethyl- piperazinomethyl)-oxazolidin-2-on;

mit 3-[4-(N-1-Naphthoylamidino)-phenyl]-5-methansulfonyloxy-methy oxazolidin-2-on das 3-[4-(N-1-Naphthoylamidino)-phenyl]-5-(4-ethoxycarbonylethyl- piperazinomethyl)-oxazolidin-2-on;

mit 3-[4-(N-2-Naphthoylamidino)-phenyl]-5-methansulfonyloxy-methyl oxazolidin-2-on das 3-[4-(N-2-Naphthoylamidino)-phenyl]-5-(4-ethoxycarbonylethyl- piperazinomethyl)-oxazolidin-2-on;

mit 3-[4-(N-2-Furoylamidino)-phenyl]-5-methansulfonyloxy-methyl-oxazolidin-2-on das 3-[4-(N-2-Furoylamidino)-phenyl]-5-(4-ethoxycarbonylethyl- piperazinomethyl)-oxazolidin-2-on;

mit 3-[4-(N-3-Furoylamidino)-phenyl]-5-methansulfonyloxy-methyl-oxazolidin-2-on das 3-[4-(N-3-Furoylamidino)-phenyl]-5-(4-ethoxycarbonylethyl- piperazinomethyl)-oxazolidin-2-on;

mit 3-[4-(N-2-Thienoylamidino)-phenyl]-5-methansulfonyloxy-methyl-oxazolidin-2-on das 3-[4-(N-2-Thienoylamidino)-phenyl]-5-(4-ethoxycarbonylethyl- piperazinomethyl)-oxazolidin-2-on;

mit 3-[4-(N-3-Thienoylamidino)-phenyl]-5-methansulfonyloxy-methyl-oxazolidin-2-on das 3-[4-(N-3-Thienoylamidino)-phenyl]-5-(4-ethoxycarbonylethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-ethoxycarbonylethyl- piperazinomethyl)-oxazolidin-2-on.

**Beispiel 3**

Analog Beispiel 1 erhält man ausgehend von 1,2 g 4-tert.-Butoxycarbonylethyl-piperazin ("C") in 20 ml DMF durch Umsetzung mit 3,0 g 3-[4-(N-Benzoylamidino)-phenyl]-5-methansulfonyloxy-methyl-oxa-zolidin-2-on [erhältlich gemäß Beispiel 1] nach Entfernung des Lösungsmittels und üblicher Aufarbeitung das 3-[4-(N-Benzoylamidino)-phenyl]-5-(4-tert.butoxycarbonylethyl-piperazinomethyl)-oxazolidin-2-on, F. 136-137°.
Analog erhält man durch Umsetzung von "C"

mit 3-[4-(N-Benzyloxycarbonylamidino)-phenyl]-5-methansulfonyloxymethyl-oxazolidin-2-on das

3-[4-(N-Benzyloxycarbonylamidino)-phenyl]-5-(4-tert.-butoxycarbonylethyl-piperazinomethyl)-oxazolidin-2-on, F. 133°;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-tert.-butoxycarbonylethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-tert.-butoxycarbonylethyl-piperazinomethyl)-oxazolidin-2-on;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4-tert.-butoxycarbonylethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-tert.-butoxycarbonylethyl-piperazinomethyl)-oxazolidin-2-on, F. 174°;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4-tert-butoxycarbonylethyl-piperazinomethyl)-oxazolidin-2-on, F. 80°;

mit 3-[4-(N-Methylsulfonylamidino)-phenyl]-5-methansulfonyloxy-methyl- oxazolidin-2-on das 3-[4-(N-Methylsulfonylamidino)-phenyl]-5-(4-tert.-butoxycarbonylethyl-piperazinomethyl)-oxazolidin-2-on, F. 205°;

mit 3-[4-(N-1-Naphthoylamidino)-phenyl]-5-methansulfonyloxy-methyl- oxazolidin-2-on das 3-[4-(N-1-Naphthoylamidino)-phenyl]-5-(4-tert.-butoxycarbonylethyl- piperazinomethyl)-oxazolidin-2-on, F. 111-113°;

mit 3-[4-(N-2-Naphthoylamidino)-phenyl]-5-methansulfonyloxy-methyl- oxazolidin-2-on das 3-[4-(N-2-Naphthoylamidino)-phenyl]-5-(4-tert.-butoxycarbonylethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-tert.-butoxycarbonylethyl-piperazinomethyl)-oxazolidin-2-on.

**Beispiel 4**

Analog Beispiel 1 erhält man ausgehend von 4-Methoxycarbonylethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-methoxycarbonylethyl-piperazinomethyl)-oxazolidin-2-on;

mit 3-[4-(N-Benzoylamidino)-phenyl]-5-methansulfonyloxy-methyl-oxazolidin-2-on das 3-[4-(N-Benzoylamidino)-phenyl]-5-(4-methoxycarbonylethyl-piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-methoxycarbonylethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-methoxycarbonyl-ethyl-piperazinomethyl)-oxazolidin-2-on;

mit 3-[4-(N-(1-Methyl-piperidyl-4-oxycarbonyl)-amidino)-phenyl]-5-methansulfonyloxy-methyl-oxazolidin-2-on das 3-[4-(N-(1-Methyl-piperidyl-4-oxycarbonyl)-amidino)-phenyl]-5-(4-methoxycarbonylethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-methoxycarbonylethyl-piperazinomethyl)-oxazolidin-2-on;

mit 3-[4-(N-Ethoxycarbonylmethylcarbamoyl-amidino)-phenyl]-5-methansulfonyloxy-methyl-oxazolidin-2-on das 3-[4-(N-Ethoxycarbonylmethylcarbamoyl-amidino)-phenyl]-5-(4-methoxycarbonylethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-methoxycarbonylethyl- piperazinomethyl)-oxazolidin-2-on;

mit 3-[4-(N-1-Naphthoylamidino)-phenyl]-5-methansulfonyloxy-methyl- oxazolidin-2-on das 3-[4-(N-1-Naphthoylamidino)-phenyl]-5-(4-methoxycarbonylethyl- piperazinomethyl)-oxazolidin-2-on;

mit 3-[4-(N-2-Naphthoylamidino)-phenyl]-5-methansulfonyloxy-methyl- oxazolidin-2-on das 3-[4-(N-2-Naphthoylamidino)-phenyl]-5-(4-methoxycarbonylethyl- piperazinomethyl)-oxazolidin-2-on;

mit 3-[4-(N-2-Furoylamidino)-phenyl]-5-methansulfonyloxy-methyl- oxazolidin-2-on das 3-[4-(N-2-Furoylamidino)-phenyl]-5-(4-methoxycarbonylethyl- piperazinomethyl)-oxazolidin-2-on;

mit 3-[4-(N-3-Furoylamidino)-phenyl]-5-methansulfonyloxy-methyl- oxazolidin-2-on das 3-[4-(N-3-Furoylamidino)-phenyl]-5-(4-methoxycarbonylethyl- piperazinomethyl)-oxazolidin-2-on;

mit 3-[4-(N-2-Thienoylamidino)-phenyl]-5-methansulfonyloxy-methyl- oxazolidin-2-on das 3-[4-(N-2-Thienoylamidino)-phenyl]-5-(4-methoxycarbonylethyl- piperazinomethyl)-oxazolidin-2-on;

mit 3-[4-(N-3-Thienoylamidino)-phenyl]-5-methansulfonyloxy-methyl- oxazolidin-2-on das 3-[4-(N-3-Thienoylamidino)-phenyl]-5-(4-methoxycarbonylethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-methoxycarbonylethyl- piperazinomethyl)-oxazolidin-2-on.

**Beispiel 5**

Analog Beispiel 1 erhält man ausgehend von 4-Isopropoxycarbonylethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-isopropoxy- carbonylethyl-piperazinomethyl)-oxazolidin-2-on;

mit 3-[4-(N-Benzoylamidino)-phenyl]-5-methansulfonyloxy-methyl- oxazolidin-2-on das 3-[4-(N-Benzoylamidino)-phenyl]-5-(4-isopropoxycarbonylethyl- piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-isopropoxycarbonylethyl-piperazinomethyl)-oxazolidin-2-on;

mit 3-[4-(N-(3-Pyridylcarbonyl)-amidino)-phenyl]-5-methansulfonyloxy methyl-oxazolidin-2-on das 3-[4-(N-(3-Pyridylcarbonyl)-amidino)-phenyl]-5-(4-isopropoxycarbonylethyl-piperazinomethyl)-oxazolidin-2-on;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4-isopropoxycarbonylethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-isopropoxycarbonylethyl-piperazinomethyl)-oxazolidin-2-on;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4-isopropoxycarbonylethyl-piperazinomethyl)-oxazolidin-2-on;

mit 3-[4-(N-Methylsulfonylamidino)-phenyl]-5-methansulfonyloxy-methyl- oxazolidin-2-on das 3-[4-(N-Methylsulfonylamidino)-phenyl]-5-(4-isopropoxycarbonylethyl-piperazinomethyl)-oxazolidin-2-on;

mit 3-[4-(N-1-Naphthoylamidino)-phenyl]-5-methansulfonyloxy-methyl- oxazolidin-2-on das 3-[4-(N-1-Naphthoylamidino)-phenyl]-5-(4-isopropoxycarbonylethyl- piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-isopropoxycarbonylethyl- piperazinomethyl)-oxazolidin-2-on;

mit 3-[4-(N-2-Furoylamidino)-phenyl]-5-methansulfonyloxy-methyl- oxazolidin-2-on das 3-[4-(N-2-Furoylamidino)-phenyl]-5-(4-isopropoxycarbonylethyl- piperazinomethyl)-oxazolidin-2-on;

mit 3-[4-(N-3-Furoylamidino)-phenyl]-5-methansulfonyloxy-methyl- oxazolidin-2-on das 3-[4-(N-3-Furoylamidino)-phenyl]-5-(4-isopropoxycarbonylethyl- piperazinomethyl)-oxazolidin-2-on;

mit 3-[4-(N-2-Thienoylamidino)-phenyl]-5-methansulfonyloxy-methyl- oxazolidin-2-on das 3-[4-(N-2-Thienoylamidino)-phenyl]-5-(4-isopropoxycarbonylethyl- piperazinomethyl)-oxazolidin-2-on;

mit 3-[4-(N-3-Thienoylamidino)-phenyl]-5-methansulfonyloxy-methyl- oxazolidin-2-on das 3-[4-(N-3-Thienoylamidino)-phenyl]-5-(4-isopropoxycarbonylethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-isopropoxycarbonylethyl-piperazinomethyl)-oxazolidin-2-on.

**Beispiel 6**

Analog Beispiel 1 erhält man ausgehend von 4-n-Butoxycarbonylethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-n-butoxycarbonylethyl-piperazinomethyl)-oxazolidin-2-on;

mit 3-[4-(N-Benzoylamidino)-phenyl]-5-methansulfonyloxy-methyl- oxazolidin-2-on das 3-[4-(N-Benzoylamidino)-phenyl]-5-(4-n-butoxycarbonylethyl- piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-n-butoxycarbonylethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-n-butoxycarbonylethyl-piperazinomethyl)-oxazolidin-2-on;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4-n-butoxycarbonylethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-n-butoxycarbonylethyl-piperazinomethyl)-oxazolidin-2-on;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4-n-butoxycarbonylethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-n-butoxycarbonylethyl-piperazinomethyl)-oxazolidin-2-on;

mit 3-[4-(N-1-Naphthoylamidino)-phenyl]-5-methansulfonyloxy-methyl-oxazolidin-2-on das 3-[4-(N-1-Naphthoylamidino)-phenyl]-5-(4-n-butoxycarbonylethyl-piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-n-butoxycarbonylethyl-piperazinomethyl)-oxazolidin-2-on;

mit 3-[4-(N-2-Furoylamidino)-phenyl]-5-methansulfonyloxy-methyl-oxazolidin-2-on das

3-[4-(N-2-Furoylamidino)-phenyl]-5-(4-n-butoxycarbonylethyl- piperazinomethyl)-oxazolidin-2-on;

mit 3-[4-(N-3-Furoylamidino)-phenyl]-5-methansulfonyloxy-methyl-oxazolidin-2-on das 3-[4-(N-3-Furoylamidino)-phenyl]-5-(4-n-butoxycarbonylethyl- piperazinomethyl)-oxazolidin-2-on;

mit 3-[4-(N-2-Thienoylamidino)-phenyl]-5-methansulfonyloxy-methyl-oxazolidin-2-on das 3-[4-(N-2-Thienoylamidino)-phenyl]-5-(4-n-butoxycarbonylethyl- piperazinomethyl)-oxazolidin-2-on;

mit 3-[4-(N-3-Thienoylamidino)-phenyl]-5-methansulfonyloxy-methyl-oxazolidin-2-on das 3-[4-(N-3-Thienoylamidino)-phenyl]-5-(4-n-butoxycarbonylethyl-piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-n-butoxycarbonylethyl-piperazinomethyl)-oxazolidin-2-on.

**Beispiel 7**

Analog Beispiel 1 erhält man ausgehend von 4-Benzyloxycarbonylethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-benzyloxycarbonylethyl-piperazinomethyl)-oxazolidin-2-on;

mit 3-[4-(N-Benzoylamidino)-phenyl]-5-methansulfonyloxy-methyl-oxazolidin-2-on das 3-[4-(N-Benzoylamidino)-phenyl]-5-(4-benzyloxycarbonylethyl-piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-benzyloxycarbonylethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-benzyloxycarbonylethyl-piperazinomethyl)-oxazolidin-2-on;

mit 3-[4-(N-(1-Methyl-piperidyl-4-oxycarbonyl)-amidino)-phenyl]-5-methansulfonyloxy-methyl-oxazolidin-2-on das 3-[4-(N-(1-Methyl-piperidyl-4-oxycarbonyl)-amidino)-phenyl]-5-(4-benzyloxycarbonylethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-benzyloxycarbonylethyl-piperazinomethyl)-oxazolidin-2-on;

mit 3-[4-(N-Ethoxycarbonylmethylcarbamoyl-amidino)-phenyl]-5-methansulfonyloxy-methyl-oxazolidin-2-on das 3-[4-(N-Ethoxycarbonylmethylcarbamoyl-amidino)-phenyl]-5-(4-benzyloxycarbonylethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-benzyloxycarbonylethyl-piperazinomethyl)-oxazolidin-2-on;

mit 3-[4-(N-1-Naphthoylamidino)-phenyl]-5-methansulfonyloxy-methyl-oxazolidin-2-on das 3-[4-(N-1-Naphthoylamidino)-phenyl]-5-(4-benzyloxycarbonylethyl-piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-benzyloxycarbonylethyl- piperazinomethyl)-oxazolidin-2-on;

mit 3-[4-(N-2-Furoylamidino)-phenyl]-5-methansulfonyloxy-methyl- oxazolidin-2-on das 3-[4-(N-2-Furoylamidino)-phenyl]-5-(4-benzyloxycarbonylethyl- piperazinomethyl)-oxazolidin-2-on;

mit 3-[4-(N-3-Furoylamidino)-phenyl]-5-methansulfonyloxy-methyl- oxazolidin-2-on das 3-[4-(N-3-Furoylamidino)-phenyl]-5-(4-benzyloxycarbonylethyl- piperazinomethyl)-oxazolidin-2-on;

mit 3-[4-(N-2-Thienoylamidino)-phenyl]-5-methansulfonyloxy-methyl- oxazolidin-2-on das 3-[4-(N-2-Thienoylamidino)-phenyl]-5-(4-benzyloxycarbonylethyl- piperazinomethyl)-oxazolidin-2-on;

mit 3-[4-(N-3-Thienoylamidino)-phenyl]-5-methansulfonyloxy-methyl- oxazolidin-2-on das 3-[4-(N-3-Thienoylamidino)-phenyl]-5-(4-benzyloxycarbonylethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-benzyloxycarbonylethyl-piperazinomethyl)-oxazolidin-2-on.

**Beispiel 8**

Analog Beispiel 1 erhält man ausgehend von 4-Methoxycarbonylmethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-methoxycarbonylmethyl-piperazinomethyl)-oxazolidin-2-on;

mit 3-[4-(N-Benzoylamidino)-phenyl]-5-methansulfonyloxy-methyl- oxazolidin-2-on das 3-[4-(N-Benzoylamidino)-phenyl]-5-(4-methoxycarbonylmethyl- piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-methoxycarbonylmethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-methoxycarbonylmethyl-piperazinomethyl)-oxazolidin-2-on;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4- methoxycarbonylmethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-methoxycarbonylmethyl-piperazinomethyl)-oxazolidin-2-on;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4-methoxycarbonylmethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-methoxycarbonylmethyl-piperazinomethyl)-oxazolidin-2-on;

mit 3-[4-(N-1-Naphthoylamidino)-phenyl]-5-methansulfonyloxy-methyl-oxazolidin-2-on das 3-[4-(N-1-Naphthoylamidino)-phenyl]-5-(4-methoxycarbonylmethyl-piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-methoxycarbonylmethyl-piperazinomethyl)-oxazolidin-2-on;

mit 3-[4-(N-2-Furoylamidino)-phenyl]-5-methansulfonyloxy-methyl- oxazolidin-2-on das 3-[4-(N-2-Furoylamidino)-phenyl]-5-(4-methoxycarbonylmethyl- piperazinomethyl)-oxazolidin-2-on;

mit 3-[4-(N-3-Furoylamidino)-phenyl]-5-methansulfonyloxy-methyl- oxazolidin-2-on das 3-[4-(N-3-Furoylamidino)-phenyl]-5-(4-methoxycarbonylmethyl- piperazinomethyl)-oxazolidin-2-on;

mit 3-[4-(N-2-Thienoylamidino)-phenyl]-5-methansulfonyloxy-methyl- oxazolidin-2-on das 3-[4-(N-2-Thienoylamidino)-phenyl]-5-(4-methoxycarbonylmethyl- piperazinomethyl)-oxazolidin-2-on;

mit 3-[4-(N-3-Thienoylamidino)-phenyl]-5-methansulfonyloxy-methyl- oxazolidin-2-on das 3-[4-(N-3-Thienoylamidino)-phenyl)-5-(4-methoxycarbonylmethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-methoxycarbonylmethyl-piperazinomethyl)-oxazolidin-2-on.

**Beispiel 9**

Analog Beispiel 1 erhält man ausgehend von 4-Isopropoxycarbonylmethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-isopropoxycarbonylmethyl-piperazinomethyl)-oxazolidin-2-on;

mit 3-[4-(N-Benzoylamidino)-phenyl]-5-methansulfonyloxy-methyl-oxazolidin-2-on das 3-[4-(N-Benzoylamidino)-phenyl]-5-(4-isopropoxycarbonylmethyl-piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-isopropoxycarbonylmethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-isopropoxycarbonylmethyl-piperazino-methyl)-oxazolidin-2-on;

mit 3-[4-(N-(1-Methyl-piperidyl-4-oxycarbonyl)-amidino)-phenyl]-5-methansulfonyloxy-methyl-oxazolidin-2-on das 3-[4-(N-(1-Methyl-piperidyl-4-oxycarbonyl)-amidino)-phenyl]-5-(4-isopropoxycarbonylmethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-isopropoxycarbonylmethyl-piperazinomethyl)-oxazolidin-2-on;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4-isopropoxycarbonylmethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-isopropoxycarbonylmethyl-piperazinomethyl)-oxazolidin-2-on;

mit 3-[4-(N-1-Naphthoylamidino)-phenyl]-5-methansulfonyloxy-methyl-oxazolidin-2-on das 3-[4-(N-1-Naphthoylamidino)-phenyl]-5-(4-isopropoxycarbonylmethyl-piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-isopropoxycarbonylmethyl-piperazinomethyl)-oxazolidin-2-on;

mit 3-[4-(N-2-Furoylamidino)-phenyl]-5-methansulfonyloxy-methyl-oxazolidin-2-on das 3-[4-(N-2-Furoylamidino)-phenyl]-5-(4-isopropoxycarbonylmethyl- piperazinomethyl)-oxazolidin-2-on;

mit 3-[4-(N-3-Furoylamidino)-phenyl]-5-methansulfonyloxy-methyl-oxazolidin-2-on das 3-[4-(N-3-Furoylamidino)-phenyl]-5-(4-isopropoxycarbonylmethyl- piperazinomethyl)-oxazolidin-2-on;

mit 3-[4-(N-2-Thienoylamidino)-phenyl]-5-methansulfonyloxy-methyl-oxazolidin-2-on das 3-[4-(N-2-Thienoylamidino)-phenyl]-5-(4-isopropoxycarbonylmethyl-piperazinomethyl)-oxazolidin-2-on;

mit 3-[4-(N-3-Thienoylamidino)-phenyl]-5-methansulfonyloxy-methyl-oxazolidin-2-on das 3-[4-(N-3-Thienoylamidino)-phenyl]-5-(4-isopropoxycarbonylmethyl-piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-isopropoxycarbonylmethyl-piperazinomethyl)-oxazolidin-2-on.

**Beispiel 10**

Analog Beispiel 1 erhält man ausgehend von 4-n-Butoxycarbonylmethylpiperazin durch Umsetzung

mit 3-[4-(N-Benzyloxycarbonylamidino)-phenyl]-5-methansulfonyloxymethyl-oxazolidin-2-on das

3-[4-(N-Benzyloxycarbonylamidino)-phenyl]-5-(4-n-butoxycarbonylmethyl-piperazinomethyl)-oxazolidin-2-on;

mit 3-[4-(N-Benzoylamidino)-phenyl]-5-methansulfonyloxy-methyl-oxazolidin-2-on das 3-[4-(N-Benzoylamidino)-phenyl]-5-(4-n-butoxycarbonylmethyl- piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-n-butoxy- carbonylmethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-n-butoxycarbonylmethyl-piperazinomethyl)-oxazolidin-2-on;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4-n-butoxycarbonylmethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-n-butoxycarbonylmethyl-piperazinomethyl)-oxazolidin-2-on;

mit 3-[4-(N-Ethoxycarbonylmethylcarbamoyl-amidino)-phenyl]-5- methansulfonyloxy-methyl-oxazolidin-2-on das 3-[4-(N-Ethoxycarbonylmethylcarbamoyl-amidino)-phenyl]-5-(4-n-butoxycarbonylmethyl-piperazinomethyl)-oxazolidin-2-on;

mit 3-[4-(N-Methylsulfonylamidino)-phenyl]-5-methansulfonyloxy-methyl- oxazolidin-2-on das 3-[4-(N-Methylsulfonylamidino)-phenyl]-5-(4-n-butoxycarbonylmethyl- piperazinomethyl)-oxazolidin-2-on;

mit 3-[4-(N-1-Naphthoylamidino)-phenyl]-5-methansulfonyloxy-methyl-oxazolidin-2-on das 3-[4-(N-1-Naphthoylamidino)-phenyl]-5-(4-n-butoxycarbonylmethyl- piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-n-butoxycarbonylmethyl-piperazinomethyl)-oxazolidin-2-on;

mit 3-[4-(N-2-Furoylamidino)-phenyl]-5-methansulfonyloxy-methyl-oxazolidin-2-on das 3-[4-(N-2-Furoylamidino)-phenyl]-5-(4-n-butoxycarbonylmethyl- piperazinomethyl)-oxazolidin-2-on;

mit 3-[4-(N-3-Furoylamidino)-phenyl]-5-methansulfonyloxy-methyl- oxazolidin-2-on das 3-[4-(N-3-Furoylamidino)-phenyl]-5-(4-n-butoxycarbonylmethyl- piperazinomethyl)-oxazolidin-2-on;

mit 3-[4-(N-2-Thienoylamidino)-phenyl]-5-methansulfonyloxy-methyl- oxazolidin-2-on das 3-[4-(N-2-Thienoylamidino)-phenyl]-5-(4-n-butoxycarbonylmethyl- piperazinomethyl)-oxazolidin-2-on;

mit 3-[4-(N-3-Thienoylamidino)-phenyl]-5-methansulfonyloxy-methyl- oxazolidin-2-on das 3-[4-(N-3-Thienoylamidino)-phenyl]-5-(4-n-butoxycarbonylmethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-n-butoxycarbonylmethyl-piperazinomethyl)-oxazolidin-2-on.

**Beispiel 11**

Analog Beispiel 1 erhält man ausgehend von 4-Benzyloxycarbonylmethylpiperazin durch Umsetzung

mit 3-[4-(N-Benzyloxycarbonylamidino)-phenyl]-5-methansulfonyloxymethyl-oxazolidin-2-on das 3-[4-(N-Benzyloxycarbonylamidino)-phenyl]-5-(4-benzyloxycarbonylmethyl-piperazinomethyl)-oxazolidin-2-on;

mit 3-[4-(N-Benzoylamidino)-phenyl]-5-methansulfonyloxy-methyl- oxazolidin-2-on das 3-[4-(N-Benzoylamidino)-phenyl]-5-(4-benzyloxycarbonylmethyl- piperazinomethyl)-oxazolidin-2-on;

mit 3-[4-(N-Phenoxycarbonylamidino)-phenyl]-5-methansulfonyloxymethyl-oxazolidin-2-on das 3-[4-(N-Phenoxycarbonylamidino)-phenyl]-5-(4-benzyloxycarbonylmethyl-piperazinomethyl)-oxazolidin-2-on;

mit 3-[4-(N-(3-Pyridylcarbonyl)-amidino)-phenyl]-5-methansulfonyloxymethyl-oxazolidin-2-on das 3-[4-(N-(3-Pyridylcarbonyl)-amidino)-phenyl]-5-(4-benzyloxycarbonylmethyl-piperazinomethyl)-oxazolidin-2-on;

mit 3-[4-(N-(1-Methyl-piperidyl-4-oxycarbonyl)-amidino)-phenyl]-5- methansulfonyloxy-methyl-oxazolidin-2-on das 3-[4-(N-(1-Methyl-piperidyl-4-oxycarbonyl)-amidino)-phenyl]-5-(4-benzyloxycarbonylmethyl-piperazinomethyl)-oxazolidin-2-on;

mit 3-[4-(N-Methoxycarbonylamidino)-phenyl]-5-methansulfonyloxymethyl-oxazolidin-2-on das 3-[4-(N-Methoxycarbonylamidino)-phenyl]-5-(4-benzyloxycarbonylmethyl-piperazinomethyl)-oxazolidin-2-on;

mit 3-[4-(N-Ethoxycarbonylmethylcarbamoyl-amidino)-phenyl]-5-methansulfonyloxy-methyl-oxazolidin-2-on das 3-[4-(N-Ethoxycarbonylmethylcarbamoyl-amidino)-phenyl]-5-(4-benzyloxycarbonylmethyl-piperazinomethyl)-oxazolidin-2-on;

mit 3-[4-(N-Methylsulfonylamidino)-phenyl]-5-methansulfonyloxy-methyl-oxazolidin-2-on das 3-[4-(N-Methylsulfonylamidino)-phenyl]-5-(4-benzyloxycarbonylmethyl-piperazinomethyl)-oxazolidin-2-on;

mit 3-[4-(N-1-Naphthoylamidino)-phenyl]-5-methansulfonyloxy-methyl-oxazolidin-2-on das 3-[4-(N-1-Naphthoylamidino)-phenyl]-5-(4-benzyloxycarbonylmethyl-piperazinomethyl)-oxazolidin-2-on;

mit 3-[4-(N-2-Naphthoylamidino)-phenyl]-5-methansulfonyloxy-methyl-oxazolidin-2-on das 3-[4-(N-2-Naphthoylamidino)-phenyl]-5-(4-benzyloxycarbonylmethyl-piperazinomethyl)-oxazolidin-2-on;

mit 3-[4-(N-2-Furoylamidino)-phenyl]-5-methansulfonyloxy-methyl-oxazolidin-2-on das 3-[4-(N-2-Furoylamidino)-phenyl]-5-(4-benzyloxycarbonylmethyl- piperazinomethyl)-oxazolidin-2-on;

mit 3-[4-(N-3-Furoylamidino)-phenyl]-5-methansulfonyloxy-methyl-oxazolidin-2-on das 3-[4-(N-3-Furoylamidino)-phenyl]-5-(4-benzyloxycarbonylmethyl- piperazinomethyl)-oxazolidin-2-on;

mit 3-[4-(N-2-Thienoylamidino)-phenyl]-5-methansulfonyloxy-methyl- oxazolidin-2-on das 3-[4-(N-2-Thienoylamidino)-phenyl]-5-(4-benzyloxycarbonylmethyl- piperazinomethyl)-oxazolidin-2-on;

mit 3-[4-(N-3-Thienoylamidino)-phenyl]-5-methansulfonyloxy-methyl- oxazolidin-2-on das 3-[4-(N-3-Thienoylamidino)-phenyl]-5-(4-benzyloxycarbonylmethyl- piperazinomethyl)-oxazolidin-2-on;

mit 3-[4-(N-Diphenylacetyl-amidino)-phenyl]-5-methansulfonyloxymethyl-oxazolidin-2-on das 3-[4-(N-Diphenylacetyl-amidino)-phenyl]-5-(4-benzyloxycarbonylmethyl-piperazinomethyl)-oxazolidin-2-on.

**Beispiel 25**

Analog Beispiel 1 erhält man durch Umsetzung von 4-Ethoxycarbonylmethyl-piperazin ("A")

mit 3-[4-(N-4-Chlorbenzoylamidino)-phenyl]-5-methansulfonyloxy-methyl-oxazolidin-2-on das 3-[4-(N-4-Chlorbenzoylamidino)-phenyl]-5-(4-ethoxycarbonylmethyl-piperazinomethyl)-oxazolidin-2-on;

mit 3-[4-(N-4-Fluorbenzoylamidino)-phenyl]-5-methansulfonyloxy-methyl-oxazolidin-2-on das 3-[4-(N-4-Fluorbenzoylamidino)-phenyl]-5-(4-ethoxycarbonylmethyl-piperazinomethyl)-oxazolidin-2-on;

mit 3-[4-(N-4-Methoxybenzoylamidino)-phenyl]-5-methansulfonyloxymethyl-oxazolidin-2-on das 3-[4-(N-4-Methoxybenzoylamidino)-phenyl]-5-(4-ethoxycarbonylmethyl-piperazinomethyl)-oxazolidin-2-on, F. 115-120°;

mit 3-[4-(N-3,4-Methylendioxybenzoylamidino)-phenyl]-5-methansulfonyloxy-methyl-oxazolidin-2-on das 3-[4-(N-3,4-Methylendioxybenzoylamidino)-phenyl]-5-(4-ethoxycarbonylmethyl-piperazinomethyl)-oxazolidin-2-on, F. 168-169°;

mit 3-[4-(N-4-Trifluormethylbenzoylamidino)-phenyl]-5-methansulfonyloxy-methyl-oxazolidin-2-on das

3-[4-(N-4-Trifluormethylbenzoylamidino)-phenyl]-5-(4-ethoxycarbonylmethyl-piperazinomethyl)-oxazolidin-2-on;

mit 3-[4-(N-4-Cyanbenzoylamidino)-phenyl]-5-methansulfonyloxy-methyl-oxazolidin-2-on das 3-[4-(N-4-Cyanbenzoylamidino)-phenyl]-5-(4-ethoxycarbonylmethyl-piperazinomethyl)-oxazolidin-2-on;

mit 3-[4-(N-4-Methoxybenzoylamidino)-phenyl]-5(R)-methansulfonyloxymethyl-oxazolidin-2-on das 3-[4-(N-4-Methoxybenzoylamidino)-phenyl]-5(R)-(4-ethoxycarbonylmethyl-piperazinomethyl)-oxazolidin-2-on, F. 153-154°, $[\alpha]_{20}^{D}$ = +31,2° (DMSO);

mit 3-[4-(N-4-Nitrobenzoylamidino)-phenyl]-5-methansulfonyloxy-methyl-oxazolidin-2-on das 3-[4-(N-4-Nitrobenzoylamidino)-phenyl]-5-(4-ethoxycarbonylmethyl-piperazinomethyl)-oxazolidin-2-on;

mit 3-[4-(N-4-Methylbenzoylamidino)-phenyl]-5-methansulfonyloxymethyl-oxazolidin-2-on das 3-[4-(N-4-Methylbenzoylamidino)-phenyl]-5-(4-ethoxycarbonylmethyl-piperazinomethyl)-oxazolidin-2-on;

mit 3-[4-(N-4-Methoxycarbonylbenzoylamidino)-phenyl]-5-methansulfonyloxy-methyl-oxazolidin-2-on das 3-[4-(N-4-Methoxycarbonylbenzoylamidino)-phenyl]-5-(4-ethoxycarbonylmethyl-piperazinomethyl)-oxazolidin-2-on;

mit 3-[4-(N-4-tert.-Butylbenzoylamidino)-phenyl]-5-methansulfonyloxymethyl-oxazolidin-2-on das 3-[4-(N-4-tert.-Butylbenzoylamidino)-phenyl]-5-(4-ethoxycarbonylmethyl-piperazinomethyl)-oxazolidin-2-on;

mit 3-[4-(N-3-Chlorbenzoylamidino)-phenyl]-5-methansulfonyloxy-methyl- oxazolidin-2-on das 3-[4-(N-3-Chlorbenzoylamidino)-phenyl]-5-(4-ethoxycarbonylmethyl- piperazinomethyl)-oxazolidin-2-on;

mit 3-[4-(N-3-Fluorbenzoylamidino)-phenyl]-5-methansulfonyloxy-methyl-oxazolidin-2-on das 3-[4-(N-3-Fluorbenzoylamidino)-phenyl]-5-(4-ethoxycarbonylmethyl-piperazinomethyl)-oxazolidin-2-on;

mit 3-[4-(N-3-Methoxybenzoylamidino)-phenyl]-5-methansulfonyloxymethyl-oxazolidin-2-on das 3-[4-(N-3-Methoxybenzoylamidino)-phenyl]-5-(4-ethoxycarbonylmethyl-piperazinomethyl)-oxazolidin-2-on;

mit 3-[4-(N-3,4-Dimethoxybenzoylamidino)-phenyl]-5-methansulfonyloxy-methyl-oxazolidin-2-on das 3-[4-(N-3,4-Dimethoxybenzoylamidino)-phenyl]-5-(4-ethoxycarbonyl-methyl-piperazino-methyl)-oxazolidin-2-on;

mit 3-[4-(N-3-Trifluormethylbenzoylamidino)-phenyl]-5(R)-methansulfonyloxy-methyl-oxazolidin-2-on das 3-[4-(N-3-Trifluormethylbenzoylamidino)-phenyl]-5(R)-(4-ethoxycarbonylmethyl-piperazinomethyl)-oxazolidin-2-on, F. 128-129°, $[\alpha]_{20}^{D}$ = +29,7° (DMSO);

mit 3-[4-(N-3-Cyanbenzoylamidino)-phenyl]-5-methansulfonyloxy-methyl-oxazolidin-2-on das 3-[4-(N-3-Cyanbenzoylamidino)-phenyl]-5-(4-ethoxycarbonylmethyl- piperazinomethyl)-oxazolidin-2-on;

mit 3-[4-(N-3-Nitrobenzoylamidino)-phenyl]-5-methansulfonyloxy-methyl-oxazolidin-2-on das 3-[4-(N-3-Nitrobenzoylamidino)-phenyl]-5-(4-ethoxycarbonylmethyl-piperazinomethyl)-oxazolidin-2-on;

mit 3-[4-(N-3-Methylbenzoylamidino)-phenyl]-5-methansulfonyloxymethyl-oxazolidin-2-on das 3-[4-(N-3-Methylbenzoylamidino)-phenyl]-5-(4-ethoxycarbonylmethyl- piperazinomethyl)-oxazolidin-2-on;

mit 3-[4-(N-3-Methoxycarbonylbenzoylamidino)-phenyl]-5-methansulfonyloxy-methyl-oxazolidin-2-on das 3-[4-(N-3-Methoxycarbonylbenzoylamidino)-phenyl]-5-(4-ethoxycarbonylmethyl-piperazinomethyl)-oxazolidin-2-on;

mit 3-[4-(N-3-tert.-Butylbenzoylamidino)-phenyl]-5-methansulfonyloxymethyl-oxazolidin-2-on das 3-[4-(N-3-tert.-Butylbenzoylamidino)-phenyl]-5-(4-ethoxycarbonylmethyl-piperazinomethyl)-oxazolidin-2-on;

mit 3-[4-(N-2-Chlorbenzoylamidino)-phenyl]-5-methansulfonyloxy-methyl-oxazolidin-2-on das 3-[4-(N-2-Chlorbenzoylamidino)-phenyl]-5-(4-ethoxycarbonylmethyl- piperazinomethyl)-oxazolidin-2-on;

mit 3-[4-(N-2-Fluorbenzoylamidino)-phenyl]-5-methansulfonyloxy-methyl- oxazolidin-2-on das 3-[4-(N-2-Fluorbenzoylamidino)-phenyl]-5-(4-ethoxycarbonylmethyl- piperazinomethyl)-oxazolidin-2-on;

mit 3-[4-(N-2-Methoxybenzoylamidino)-phenyl]-5-methansulfonyloxymethyl-oxazolidin-2-on das 3-[4-(N-2-Methoxybenzoylamidino)-phenyl]-5-(4-ethoxycarbonylmethyl-piperazinomethyl)-oxazolidin-2-on;

mit 3-[4-(N-2,3,4-Trimethoxybenzoylamidino)-phenyl]-5-methansulfonyloxy-methyl-oxazolidin-2-on das 3-[4-(N-2,3,4-Trimethoxybenzoylamidino)-phenyl]-5-(4-ethoxycarbonylmethyl-piperazinomethyl)-oxazolidin-2-on;

mit 3-[4-(N-2-Trifluormethylbenzoylamidino)-phenyl]-5-methansulfonyloxy-methyl-oxazolidin-2-on das 3-[4-(N-2-Trifluormethylbenzoylamidino)-phenyl]-5-(4-ethoxycarbonylmethyl-piperazinomethyl)-oxazolidin-2-on, F. 121°;

mit 3-[4-(N-2-Cyanbenzoylamidino)-phenyl]-5-methansulfonyloxy-methyl-oxazolidin-2-on das 3-[4-(N-2-Cyanbenzoylamidino)-phenyl]-5-(4-ethoxycarbonylmethyl-piperazinomethyl)-oxazolidin-2-on;

mit 3-[4-(N-2-Nitrobenzoylamidino)-phenyl]-5-methansulfonyloxy-methyl-oxazolidin-2-on das 3-[4-(N-2-Nitrobenzoylamidino)-phenyl]-5-(4-ethoxycarbonylmethyl- piperazinomethyl)-oxazolidin-2-on;

mit 3-[4-(N-2-Methylbenzoylamidino)-phenyl]-5-methansulfonyloxymethyl-oxazolidin-2-on das 3-[4-(N-2-Methylbenzoylamidino)-phenyl]-5-(4-ethoxycarbonylmethyl-piperazinomethyl)-oxazolidin-2-on;

mit 3-[4-(N-2-Methoxycarbonylbenzoylamidino)-phenyl]-5-methansulfonyloxy-methyl-oxazolidin-2-on das 3-[4-(N-2-Methoxycarbonylbenzoylamidino)-phenyl]-5-(4-ethoxycarbonylmethyl-piperazinomethyl)-oxazolidin-2-on;

mit 3-[4-(N-2-tert.-Butylbenzoylamidino)-phenyl]-5-methansulfonyloxymethyl-oxazolidin-2-on das 3-[4-(N-2-tert.-Butylbenzoylamidino)-phenyl]-5-(4-ethoxycarbonylmethyl-piperazinomethyl)-oxazolidin-2-on.

**Beispiel 27**

Analog Beispiel 1 erhält man durch Umsetzung von 4-Ethoxycarbonylmethyl-piperazin ("A")

mit 3-[4-(N-2-Acetoxybenzoylamidino)-phenyl]-5-methansulfonyloxymethyl-oxazolidin-2-on das 3-[4-(N-2-Acetoxybenzoylamidino)-phenyl]-5-(4-ethoxycarbonylmethyl-piperazinomethyl)-oxazolidin-2-on.

**Beispiel 28**

Analog Beispiel 1 erhält man durch Umsetzung von 4-Ethoxycarbonylethyl-piperazin

mit 3-[4-(N-2-Acetoxybenzoylamidino)-phenyl]-5-methansulfonyloxymethyl-oxazolidin-2-on das 3-[4-(N-2-Acetoxybenzoylamidino)-phenyl]-5-(4-ethoxycarbonylethyl-piperazinomethyl)-oxazolidin-2-on.

**Beispiel 31**

Analog Beispiel 1 erhält man durch Umsetzung von 4-(2-Acetoxyphenoxycarbonylmethyl)-piperazin

mit 3-[4-(N-2-Acetoxybenzoylamidino)-phenyl]-5-methansulfonyloxymethyl-oxazolidin-2-on das 3-[4-(N-2-Acetoxybenzoylamidino)-phenyl]-5-[4-(2-acetoxyphenoxycarbonylmethyl)-piperazinomethyl]-oxazolidin-2-on.

**Beispiel 32**

Analog Beispiel 1 erhält man durch Umsetzung von 4-(2-Acetoxyphenoxycarbonylethyl)-piperazin

mit 3-[4-(N-2-Acetoxybenzoylamidino)-phenyl]-5-methansulfonyloxymethyl-oxazolidin-2-on das 3-[4-(N-2-Acetoxybenzoylamidino)-phenyl]-5-[4-(2-acetoxyphenoxycarbonylethyl)-piperazinomethyl]-oxazolidin-2-on.

**Beispiel 33**

Analog Beispiel 1 erhält man durch Umsetzung von 4-(2-Acetoxyphenoxycarbonylmethyl)-piperazin

mit 3-[4-(N-Benzoylamidino)-phenyl]-5-methansulfonyloxy-methyl- oxazolidin-2-on das 3-[4-(N-Benzoyl-amidino)-phenyl]-5-[4-(2-acetoxyphenoxy-carbonylmethyl)-piperazinomethyl]-oxazolidin-2-on.

**Beispiel 34**

Analog Beispiel 1 erhält man durch Umsetzung von 4-(2-Acetoxyphenoxycarbonylethyl)-piperazin

mit 3-[4-(N-Benzoylamidino)-phenyl]-5-methansulfonyloxy-methyl- oxazolidin-2-on das 3-[4-(N-Benzoyl-amidino)-phenyl]-5-[4-(2-acetoxyphenoxycarbonylethyl)-piperazinomethyl]-oxazolidin-2-on.

**Beispiel 35**

Analog Beispiel 1 erhält man durch Umsetzung von 4-Ethoxycarbonylethyl-piperazin

mit 3-[4-(N-4-Chlorbenzoylamidino)-phenyl]-5-methansulfonyloxy-methyl-oxazolidin-2-on das 3-[4-(N-4-Chlorbenzoylamidino)-phenyl]-5-(4-ethoxycarbonylethyl- piperazinomethyl)-oxazolidin-2-on;

mit 3-[4-(N-4-Fluorbenzoylamidino)-phenyl]-5-methansulfonyloxy-methyl- oxazolidin-2-on das 3-[4-(N-4-Fluorbenzoylamidino)-phenyl]-5-(4-ethoxycarbonylethyl- piperazinomethyl)-oxazolidin-2-on;

mit 3-[4-(N-4-Methoxybenzoylamidino)-phenyl]-5-methansulfonyloxymethyl-oxazolidin-2-on das 3-[4-(N-4-Methoxybenzoylamidino)-phenyl]-5-(4-ethoxycarbonylethyl-piperazinomethyl)-oxazolidin-2-on, F. 147-150°;

mit 3-[4-(N-3,4-Methylendioxybenzoylamidino)-phenyl]-5-methansulfonyloxy-methyl-oxazolidin-2-on das 3-[4-(N-3,4-Methylendioxybenzoylamidino)-phenyl]-5-(4-ethoxycarbonylethyl-piperazinomethyl)-oxazolidin-2-on;

mit 3-[4-(N-4-Trifluormethylbenzoylamidino)-phenyl]-5-methansulfonyloxy-methyl-oxazolidin-2-on das 3-[4-(N-4-Trifluormethylbenzoylamidino)-phenyl]-5-(4-ethoxycarbonylethyl-piperazinomethyl)-oxazolidin-2-on, F. 187°;

mit 3-[4-(N-4-Cyanbenzoylamidino)-phenyl]-5-methansulfonyloxy-methyl-oxazolidin-2-on das 3-[4-(N-4-Cyanbenzoylamidino)-phenyl]-5-(4-ethoxycarbonylethyl-piperazinomethyl)-oxazolidin-2-on;

mit 3-[4-(N-4-Nitrobenzoylamidino)-phenyl]-5-methansulfonyloxy-methyl-oxazolidin-2-on das 3-[4-(N-4-Nitrobenzoylamidino)-phenyl]-5-(4-ethoxycarbonylethyl-piperazinomethyl)-oxazolidin-2-on;

mit 3-[4-(N-4-Methylbenzoylamidino)-phenyl]-5-methansulfonyloxymethyl-oxazolidin-2-on das 3-[4-(N-4-Methylbenzoylamidino)-phenyl]-5-(4-ethoxycarbonylethyl-piperazinomethyl)-oxazolidin-2-on;

mit 3-[4-(N-4-Methoxycarbonylbenzoylamidino)-phenyl]-5-methansulfonyloxy-methyl-oxazolidin-2-on das 3-[4-(N-4-Methoxycarbonylbenzoylamidino)-phenyl]-5-(4-ethoxycarbonylethyl-piperazinomethyl)-oxazolidin-2-on;

mit 3-[4-(N-4-tert.-Butylbenzoylamidino)-phenyl]-5-methansulfonyloxymethyl-oxazolidin-2-on das 3-[4-(N-4-tert.-Butylbenzoylamidino)-phenyl]-5-(4-ethoxycarbonylethyl-piperazinomethyl)-oxazolidin-2-on;

mit 3-[4-(N-3-Chlorbenzoylamidino)-phenyl]-5-methansulfonyloxy-methyl-oxazolidin-2-on das 3-[4-(N-3-Chlorbenzoylamidino)-phenyl]-5-(4-ethoxycarbonylethyl-piperazinomethyl)-oxazolidin-2-on;

mit 3-[4-(N-3-Fluorbenzoylamidino)-phenyl]-5-methansulfonyloxy-methyl-oxazolidin-2-on das 3-[4-(N-3-Fluorbenzoylamidino)-phenyl]-5-(4-ethoxycarbonylethyl-piperazinomethyl)-oxazolidin-2-on;

mit 3-[4-(N-3-Methoxybenzoylamidino)-phenyl]-5-methansulfonyloxymethyl-oxazolidin-2-on das 3-[4-(N-3-Methoxybenzoylam idino)-phenyl]-5-(4-ethoxycarbonylethyl-piperazinomethyl)-oxazolidin-2-on;

mit 3-[4-(N-3,4-Dimethoxybenzoylamidino)-phenyl]-5-methansulfonyloxymethyl-oxazolidin-2-on das 3-[4-(N-3,4-Dimethoxybenzoylamidino)-phenyl]-5-(4-ethoxycarbonylethyl-piperazinomethyl)-oxazolidin-2-on;

mit 3-[4-(N-3-Trifluormethylbenzoylamidino)-phenyl]-5-methansulfonyloxy-methyl-oxazolidin-2-on das 3-[4-(N-3-Trifluormethylbenzoylamidino)-phenyl]-5-(4-ethoxycarbonylethyl-piperazinomethyl)-oxazolidin-2-on, 130-131°;

mit 3-[4-(N-3-Cyanbenzoylamidino)-phenyl]-5-methansulfonyloxy-methyl-oxazolidin-2-on das 3-[4-(N-3-Cyanbenzoylamidino)-phenyl]-5-(4-ethoxycarbonylethyl-piperazinomethyl)-oxazolidin-2-on;

mit 3-[4-(N-3-Nitrobenzoylamidino)-phenyl]-5-methansulfonyloxy-methyl-oxazolidin-2-on das 3-[4-(N-3-Nitrobenzoylamidino)-phenyl]-5-(4-ethoxycarbonylethyl-piperazinomethyl)-oxazolidin-2-on;

mit 3-[4-(N-3-Methylbenzoylamidino)-phenyl]-5-methansulfonyloxymethyl-oxazolidin-2-on das 3-[4-(N-3-Methylbenzoylamidino)-phenyl]-5-(4-ethoxycarbonylethyl-piperazinomethyl)-oxazolidin-2-on;

mit 3-[4-(N-3-Methoxycarbonylbenzoylamidino)-phenyl]-5-methansulfonyloxy-methyl-oxazolidin-2-on das 3-[4-(N-3-Methoxycarbonylbenzoylamidino)-phenyl]-5-(4-ethoxycarbonylethyl-piperazinomethyl)-oxazolidin-2-on;

mit 3-[4-(N-3-tert.-Butylbenzoylamidino)-phenyl]-5-methansulfonyloxymethyl-oxazolidin-2-on das 3-[4-(N-3-tert.-Butylbenzoylamidino)-phenyl]-5-(4-ethoxycarbonylethyl-piperazinomethyl)-oxazolidin-2-on;

mit 3-[4-(N-2-Chlorbenzoylamidino)-phenyl]-5-methansulfonyloxy-methyl-oxazolidin-2-on das 3-[4-(N-2-Chlorbenzoylamidino)-phenyl]-5-(4-ethoxycarbonylethyl-piperazinomethyl)-oxazolidin-2-on;

mit 3-[4-(N-2-Fluorbenzoylamidino)-phenyl]-5-methansulfonyloxy-methyl-oxazolidin-2-on das 3-[4-(N-2-Fluorbenzoylamidino)-phenyl]-5-(4-ethoxycarbonylethyl-piperazinomethyl)-oxazolidin-2-on;

mit 3-[4-(N-2-Methoxybenzoylamidino)-phenyl]-5-methansulfonyloxymethyl-oxazolidin-2-on das 3-[4-(N-2-Methoxybenzoylamidino)-phenyl]-5-(4-ethoxycarbonylethyl-piperazinomethyl)-oxazolidin-2-on;

mit 3-[4-(N-2,3,4-Trimethoxybenzoylamidino)-phenyl]-5-methansulfonyloxy-methyl-oxazolidin-2-on das 3-[4-(N-2,3,4-Trimethoxybenzoylamidino)-phenyl]-5-(4-ethoxycarbonylethyl-piperazino-methyl)-oxazolidin-2-on;

mit 3-[4-(N-2-Trifluormethylbenzoylamidino)-phenyl]-5-methansulfonyloxy-methyl-oxazolidin-2-on das 3-[4-(N-2-Trifluormethylbenzoylamidino)-phenyl]-5-(4-ethoxycarbonylethyl-piperazinomethyl)-oxazolidin-2-on;

mit 3-[4-(N-2-Cyanbenzoylamidino)-phenyl]-5-methansulfonyloxy-methyl-oxazolidin-2-on das 3-[4-(N-2-Cyanbenzoylamidino)-phenyl]-5-(4-ethoxycarbonylethyl-piperazinomethyl)-oxazolidin-2-on;

mit 3-[4-(N-2-Nitrobenzoylamidino)-phenyl]-5-methansulfonyloxy-methyl-oxazolidin-2-on das 3-[4-(N-2-Nitrobenzoylamidino)-phenyl]-5-(4-ethoxycarbonylethyl-piperazinomethyl)-oxazolidin-2-on;

mit 3-[4-(N-2-Methylbenzoylamidino)-phenyl]-5-methansulfonyloxymethyl-oxazolidin-2-on das 3-[4-(N-2-Methylbenzoylamidino)-phenyl]-5-(4-ethoxycarbonylethyl-piperazinomethyl)-oxazolidin-2-on;

mit 3-[4-(N-2-Methoxycarbonylbenzoylamidino)-phenyl]-5-methansulfonyloxy-methyl-oxazolidin-2-on das 3-[4-(N-2-Methoxycarbonylbenzoylamidino)-phenyl]-5-(4-ethoxycarbonylethyl-piperazinomethyl)-oxazolidin-2-on;

mit 3-[4-(N-2-tert.-Butylbenzoylamidino)-phenyl]-5-methansulfonyloxymethyl-oxazolidin-2-on das 3-[4-(N-2-tert.-Butylbenzoylamidino)-phenyl]-5-(4-ethoxycarbonylethyl-piperazinomethyl)-oxazolidin-2-on

**Beispiel 36**

Analog Beispiel 1 erhält man ausgehend von 4-tert.-Butoxycarbonylmethyl-piperazin durch Umsetzung

mit 3-[4-(N-Benzoylamidino)-phenyl]-5(R)-methansulfonyloxy-methyl-oxazolidin-2-on das 3-[4-(N-Benzoylamidino)-phenyl]-5(R)-(4-tert-butoxycarbonylmethyl-piperazinomethyl)-oxazolidin-2-on, F. 160°, $[\alpha]_{20}^{D}$ = +32,7°;

mit 3-[4-(N-Benzoylamidino)-phenyl]-5-methansulfonyloxy-methyl-oxazolidin-2-on das 3-[4-(N-Benzoylamidino)-phenyl]-5-(4-tert.-butoxycarbonylmethyl-piperazinomethyl)-oxazolidin-2-on, F. 182°.

**Beispiel 38**

Analog Beispiel 1 erhält man ausgehend von 1-Ethoxycarbonylmethyl-piperazin durch Umsetzung

mit 3-[4-(N-Ethoxycarbonylamidino)-phenyl]-5(R)-chlormethyl-oxazolidin-2-on das 3-[4-(N-Ethoxycarbonylamidino)-phenyl]-5(R)-(4-ethoxycarbonylmethyl-piperazinomethyl)-oxazolidin-2-on, F. 142-143°;

mit 3-[4-(N-Ethoxycarbonylamidino)-phenyl]-5(S)-chlormethyl-oxazolidin-2-on das 3-[4-(N-Ethoxycarbonylamidino)-phenyl]-5(S)-(4-ethoxycarbonylmethyl-piperazinomethyl)-oxazolidin-2-on, F. 142-143°;

mit 3-[4-(N-Isopropoxycarbonylamidino)-phenyl]-5(R)-chlormethyl-oxazolidin-2-on das 3-[4-(N-Isopropoxycarbonylamidino)-phenyl]-5(R)-(4-ethoxycarbonylmethyl-piperazinomethyl)-oxazolidin-2-on, F. 129-130°, $[\alpha]_{20}^{D}$ = +31,2° (DMSO);

mit 3-[4-(N-Isopropoxycarbonylamidino)-phenyl]-5(S)-chlormethyl-oxazolidin-2-on das 3-[4-(N-Isopropoxycarbonylamidino)-phenyl]-5(S)-(4-ethoxycarbonylmethyl-piperazinomethyl)-oxazolidin-2-on;

mit 3-[4-(N-Methoxycarbonylamidino)-phenyl]-5(R)-chlormethyl-oxazolidin-2-on das 3-[4-(N-Methoxycarbonylamidino)-phenyl]-5(R)-(4-ethoxycarbonylmethyl-piperazinomethyl)-oxazolidin-2-on, F. 175-176°, $[\alpha]_{20}^{D}$ = +51 ° (Methanol);

mit 3-[4-(N-Methoxycarbonylamidino)-phenyl]-5(S)-chlormethyl-oxazolidin-2-on das 3-[4-(N-Methoxycarbonylamidino)-phenyl]-5(S)-(4-ethoxycarbonylmethyl-piperazinomethyl)-oxazolidin-2-on;

Analog erhält man ausgehend von 1-tert.-Butoxycarbonylmethyl-piperazin durch Umsetzung

mit 3-[4-(N-Methoxycarbonylamidino)-phenyl]-5-chlormethyl-oxazolidin-2-on das 3-[4-(N-Methoxycarbonylamidino)-phenyl]-5-(4-tert.-butoxycarbonylmethyl-piperazinomethyl)-oxazolidin-2-on, F. 181°;

mit 3-[4-(N-Ethoxycarbonylamidino)-phenyl]-5-chlormethyl-oxazolidin-2-on das 3-[4-(N-Ethoxycarbonylamidino)-phenyl]-5-(4-tert.-butoxycarbonylmethyl-piperazinomethyl)-oxazolidin-2-on.

Die nachfolgenden Beispiele betreffen pharmazeutische Zubereitungen:

**Beispiel A: Injektionsgläser**

Eine Lösung von 100 g eines Wirkstoffes der Formel 1 und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2\ H_2O$, 28,48 g $Na_2HPO_4 \cdot 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

**1.** Verbindungen der Formel I

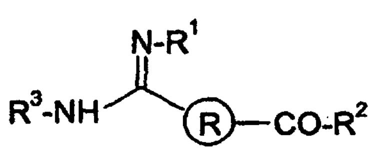

I,

worin

R

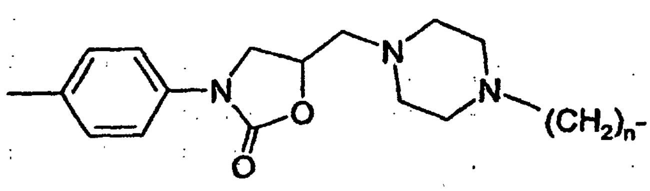

n 1 oder 2,

$R^1$ H,

$R^2$ OA oder OAr,

$R^3$ A-CO, Ar-CO, Het-CO, Het-O-CO, Ar-O-CO, A-O-CO, Ar-$SO_2$ oder A-$SO_2$,

A Alkyl mit 1 bis 6 C-Atomen,

Ar unsubstituiertes oder ein-, zwei- oder dreifach durch A, F, Cl, Br, I, OA, -O-$CH_2$-O-, COOA, COOH, $CF_3$, OH, $NO_2$, CN, $NH_2$, O-CO-A, NHA oder $NA_2$ substituiertes Aryl mit 6 bis 10 C-Atomen Diphenylmethyl oder Benzyl und

Het einen ein- oder zweikernigen gesättigten, ungesättigten oder aromatischen Heterozyklus mit 1 bis 4 N-, O- und/oder S- Atomen, der unsubstituiert oder einfach durch F, Cl, Br, $CF_3$, A, OH, OA, CN oder $NO_2$ substituiert sein kann,

bedeuten,
sowie deren physiologisch unbedenkliche Salze.

**2.** (a) 3-p-(N-Benzoylamidino)-phenyl-5-[4-(ethoxycarbonylmethyl)-piperazinomethyl]-oxazolidin-2-on,

(b) 3-p-(3-Pyridylcarbonylamidino)-phenyl-5-[4-(ethoxycarbonyl-methyl)-piperazinomethyl]-oxazolidin-2-on,

(c) 3-p-(N-Methyl-4-piperidyloxycarbonylamidino)-phenyl-5-[4-(ethoxycarbonylmethyl)-piperazinomethyl]-oxazolidin-2-on,

(d) 3-p-[N-(Ethoxycarbonylmethylcarbamoyl)-amidino]-phenyl-5-[4-(tert butoxycarbonylethyl)-piperazinomethyl]-oxazolidin-2-on,

(e) 3-p-(N-Methylsulfonylamidino)-phenyl-5-[4-(tert.-butoxycarbonylethyl)-piperazinomethyl]-oxazolidin-2-on,

(f) 3-(4-(N-Benzoylamidino)-phenyl]-5(R)-(4-ethoxycarbonylmethylpiperazinomethyl)-oxazolidin-2-on,

(g) 3-(4-(N-Benzoylamidino)-phenyl]-5(S)-(4-ethoxycarbonylmethylpiperazinomethyl)-oxazolidin-2-on,

(h) 3-(4-(N-Benzoylamidino)-phenyl]-5-(4-ethoxycarbonylmethylpiperazinomethyl)-oxazolidin-2-on,

(i) 3-(4-(N-Phenoxycarbonylamidino)-phenyl]-5-(4-ethoxycarbonylmethylpiperazinomethyl)-oxazolidin-2-on,

(j) 3-(4-(N-1-Naphthoylamidino)-phenyl]-5-(4-ethoxycarbonylmethylpiperazinomethyl)-oxazolidin-2-on,

(k) 3-(4-(N-Methoxycarbonyl-amidino)-phenyl]-5-(4-ethoxycarbonylmethylpiperazinomethyl)-oxazolidin-2-on,

(l) 3-(4-(N-Benzoylamidino)-phenyl]-5-(4-ethoxycarbonylethylpiperazinomethyl)-oxazolidin-2-on,

(m) 3-(4-(N-Benzoylamidino)-phenyl]-5(S)-(4-ethoxycarbonylethylpiperazinomethyl)-oxazolidin-2-on,

(n) 3-(4-(N-Benzoylamidino)-phenyl]-5(R)-(4-ethoxycarbonylethylpiperazinomethyl)-oxazolidin-2-on,

(o) 3-(4-(N-Benzoyloxycarbonylamidino)-phenyl]-(4-ethoxycarbonylethylpiperazinomethyl)-oxazolidin-2-on,

(p) 3-(4-(N-Benzoyloxycarbonylamidino)-phenyl]-5(R)-(4-ethoxycarbonylethylpiperazinomethyl)-oxazolidin-2-on,

(q) 3-(4-(N-Methoxycarbonylamidino)-phenyl]-5-(4-ethoxycarbonylethylpiperazinomethyl)-oxazolidin-2-on,

(r) 3-(4-(N-Benzoylamidino)-phenyl]-5-(4-tert.-butoxycarbonylethylpiperazinomethyl)-oxazolidin-2-on,

(s) 3-(4-(N-Benzoylcarbonylamidino)-phenyl]-5-(4-tert.-butoxycarbonylethylpiperazinomethyl)-oxazolidin-2-on,

(t) 3-(4-(N-Methoxycarbonylamidino)-phenyl]-5-(4-tert.-butoxycarbonylethylpiperazinomethyl)-oxazolidin-2-on,

(u) 3-(4-(N-Methoxybenzoylamidino)-phenyl]-5-(4-butoxycarbonylmethylpiperazinomethyl)-oxazolidin-2-on,

(v) 3-(4-(N-Methylendioxybenzoylamidino)-phenyl]-5-(4-ethoxycarbonylmethylpiperazinomethyl)-oxazolidin-2-on,

(w) 3-(4-(N-Methoxybenzoylamidino)-phenyl]-5(R)-(4-ethoxycarbonylmethylpiperazinomethyl)-oxazolidin-2-on,

(x) 3-(4-(N-3-Trifluormethylbenzoylamidino)-phenyl]-5(R)-(4-ethoxycarbonylmethylpiperazinomethyl)-oxazolidin-2-on,

(y) 3-(4-(N-2-Trifluormethylbenzoylamidino)-phenyl]-5-(4-ethoxycarbonylmethylpiperazinomethyl)-oxazolidin-2-on,

(z) 3-(4-(N-4-Methoxybenzoylamidino)-phenyl]-5-(4-ethoxycarbonylethylpiperazinomethyl)-oxazolidin-2-on,

(aa) 3-(4-(N-4-Trifluormethylbenzoylamidino)-phenyl]-5-(4-ethoxycarbonylethylpiperazinomethyl)-oxazolidin-2-on,

(bb) 3-(4-(N-3-Trifluormethylbenzoylamidino)-phenyl]-5-(4-ethoxycarbonylethylpiperazinomethyl)-oxazolidin-2-on,

(cc) 3-(4-(N-Benzoylamidino)-phenyl]-5-(4-tert.-butoxycarbonylmethylpiperazinomethyl)-oxazolidin-2-on,

(dd) 3-(4-(N-Ethoxycarbonylamidino)-phenyl]-5(R)-(4-ethoxycarbonylmethylpiperazinomethyl)-oxazolidin-2-on,

(ee) 3-(4-(N-Ethoxycarbonylamidino)-phenyl]-5(S)-(4-ethoxycarbonylmethylpiperazinomethyl)-oxazolidin-2-on,

(ff) 3-(4-(N-Isopropoxycarbonylamidino)-phenyl]-5(R)-(4-ethoxycarbonylmethylpiperazinomethyl)-oxazolidin-2-on,

(gg) 3-(4-(N-Methoxycarbonylamidino)-phenyl]-5(R)-(4-ethoxycarbonylmethylpiperazinomethyl)-oxazolidin-2-on,

(hh) 3-(4-(N-Methoxycarbonylamidino)-phenyl]-5-(4-tert.-butoxycarbonylmethylpiperazinomethyl)-oxazolidin-2-on,

gemäß Anspruch 1, sowie deren physiologisch unbedenklichen Salze.

**3.** Verfahren zur Herstellung einer pharmazeutischen Zubereitung, **dadurch gekennzeichnet, daß** man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

**4.** Pharmazeutische Zubereitung, **gekennzeichnet durch** einen Gehalt an mindestens einer Verbindung der Formel I, gemäß Anspruch 1, und/oder einem ihrer physiologisch unbedenklichen Salze.

**5.** Verwendung einer Verbindung der Formel I gemäß Anspruch 1 und/oder eines ihrer physiologisch unbedenklichen Salze zur Herstellung von Arzneimitteln.

**6.** Verwendung einer Verbindung der Formel I gemäß Anspruch 1 und/oder eines ihrer physiologisch unbedenklichen Salze zur Herstellung eines Arzneimittels zur Behandlung von Krankheiten.

**7.** Verwendung nach Anspruch 6 zur Behandlung von Thrombosen, Herzinfarkt und/oder Tumoren.

**Claims**

**1.** Compounds of the formula I

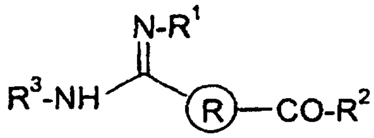

I,

in which

R is

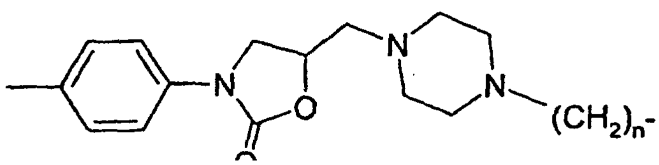

n is 1 or 2,

$R^1$ is H,

$R^2$ is OA or OAr,

$R^3$ is A-CO, Ar-CO, Het-CO, Het-O-CO, Ar-O-CO, A-O-CO, Ar- $SO_2$ or A-$SO_2$,

A is alkyl having from 1 to 6 carbon atoms,

Ar is aryl having from 6 to 10 carbon atoms which is unsubstituted or mono-, di- or trisubstituted by A, F, Cl, Br, I, OA, -O-$CH_2$-O-, COOA, COOH, $CF_3$, OH, $NO_2$, CN, $NH_2$, O- CO-A, NHA or $NA_2$, diphenylmethyl or benzyl and

Het is a mono- or binuclear saturated, unsaturated or aromatic heterocycle having from 1 to 4 N, O and/or S atoms, which may be unsubstituted or monosubstituted by F, Cl, Br, $CF_3$, A, OH, OA, CN or $NO_2$,

and physiologically acceptable salts thereof.

**2.** (a) 3-p-(N-benzoylamidino)phenyl-5-[4-(ethoxycarbonylmethyl)-piperazinomethyl}oxazolidin-2-one,

(b) 3-p-(3-pyridylcarbonylamidino)phenyl-5-[4-(ethoxycarbonylmethyl)piperazinomethyl]oxazolidin-2-one,

(c) 3-p-(N-methyl-4-piperidyloxycarbonylamidino)phenyl-5-[4-(ethoxycarbonylmethyl)piperazinomethyl]oxazolidin-2-one.

(d) 3-p-[N-(ethoxycarbonylmethylcarbamoyl)amidino}phenyl-5-[4-(tert-butoxycarbonylethyl)piperazinomethyl]oxazolidin-2-one.

(e) 3-p-(N-methylsulfonylamidino)phenyl-5-[4-(tert-butoxycarbonylethyl)piperazinomethyl]oxazolidin-2-one,

(f) 3-(4-(N-benzoylamidino)phenyl]-5(R)-(4-ethoxycarbonylmethylpiperazinomethyl)oxazolidin-2-one,

(g) 3-(4-(N-benzoylamidino)phenyl]-5(S)-(4-ethoxycarbonylmethylpiperazinomethyl)oxazolidin-2-one,

(h) 3-(4-(N-benzoylamidino)phenyl]-5-(4-ethoxycarbonylmethylpiperazinomethyl)oxazolidin-2-one,

(l) 3-(4-(N-phenoxycarbonylamidino)phenyl]-5-(4-ethoxycarbonylmethylpiperazinomethyl)oxazolidin-2-one,

(j) 3-(4-(N-1-naphthoylamidino)phenyl]-5-(4-ethoxycarbonylmethylpiperazinomethyl)oxazolidin-2-one,

(k) 3-(4-(N-methoxycarbonylamidino)phenyl)-5-(4-ethoxycarbonylmethylpiperazinomethyl)oxazolidin-2-one,

(l) 3-(4-(N-benzoylamidino)phenyl]-5-(4-ethoxycarbonylethylpiperazinomethyl)oxazolidin-2-one,

(m) 3-(4-(N-benzoylamidino)phenyl]-5(S)-(4-ethoxycarbonylethylpiperazinomethyl)oxazolidin-2-one,

(n) 3-(4-(N-benzoylamidino)phenyl]-5(R)-(4-ethoxycarbonylethylpiperazinomethyl)oxazolidin-2-one,

(o) 3-(4-(N-benzoyloxycarbonylamidino)phenyl]-(4-ethoxycarbonylethylpiperazinomethyl)oxazolidin-2-one,

(p) 3-(4-(N-benzoyloxycarbonylamidino)phenyl]-5(R)-(4-ethoxycarbonylethylpiperazinomethyl)oxazolidin-2-one,

(q) 3-(4-(N-methoxycarbonylamidino)phenyl]-5-(4-ethoxycarbonylethylpiperazinomethyl)oxazolidin-2-one,

(r) 3-(4-(N-benzoylamidino)phenyl]-5-(4-tert-butoxycarbonylethylpiperazinomethyl)oxazolidin-2-one,

(s) 3-(4-(N-benzoy!carbonylamidino)phenyl]-5-(4-tert-butoxycarbonylethylpiperazinomethyl)oxazolidin-2-one,

(t) 3-(4-(N-methoxycarbonylamidino)phenyl]-5-(4-tert-butoxycarbonylethylpiperazinomethyl)oxazolidin-2-one,

(u) 3-(4-(N-methoxybenzoylamidino)phenyl]-5-(4-butoxycarbonylmethylpiperazinomethyl)oxazolidin-2-one,

(v) 3-(4-(N-methylenedioxybenzoylamidino)phenyl]-5-(4-ethoxycarbonylmethylpiperazinomethyl)oxazolidin-2-one,

(w) 3-(4-(N-methoxybenzoylamidino)phenyl]-5(R)-(4-ethoxycarbonylmethylpiperazinomethyl)oxazolidin-2-one,

(x) 3-(4-(N-3-trifluoromethylbenzoylamidino)phenyl]-5(R)-(4-ethoxycarbonylmethylpiperazinomethyl)oxazolidin-2-one,

(y) 3-(4-(N-2-trifluoromethylbenzoylamidino)phenyl]-5-(4-ethoxycarbonylmethylpiperazinomethyl)oxazolidin-2-one,

(z) 3-(4-(N-4-methoxybenzoylamidino)phenyl]-5-(4-ethoxycarbonylethylpiperazinomethyl)oxazolidin-2-one,

(aa) 3-(4-(N-4-trifluoromethylbenzoylamidino)phenyl]-5-(4-ethoxycarbonylethylpiperazinomethyl)oxazolidin-2-one,

(bb) 3-(4-(N-3-trifluoromethylbenzoylamidino)phenyl]-5-(4-ethoxycarbonylethylpiperazinomethyl)oxazolidin-2-one,

(cc) 3-(4-(N-benzoylamidino)phenyl]-5-(4-tert-butoxycarbonylmethylpiperazinomethyl)oxazolidin-2-one,

(dd) 3-(4-(N-ethoxycarbonylamidino)phenyl]-5(R)-(4-ethoxycarbonylmethylpiperazinomethyl)oxazolidin-2-one,

(ee) 3-(4-(N-ethoxycarbonylamidino)phenyl]-5(S)-(4-ethoxycarbonylmethylpiperazinomethyl)oxazolidin-2-one,

(ff) 3-(4-(N-isopropoxycarbonylamidino)phenyl]-5(R)-(4-ethoxycarbonylmethylpiperazinomethyl)oxazolidin-2-one,

(gg) 3-(4-(N-methoxycarbonylamidino)phenyl]-5(R)-(4-ethoxycarbonylmethylpiperazinomethyl)oxazolidin-2-one,

(hh) 3-(4-(N-methoxycarbonylamidino)phenyl]-5-(4-tert-butoxycarbonylmethylpiperazinomethyl)oxazolidin-2-one,

according to Claim 1, and physiologically acceptable salts thereof.

3. Process for the preparation of a pharmaceutical preparation, **characterised in that** a compound of the formula I according to Claim 1 and/or one of its physiologically acceptable salts is brought into a suitable administration form together with at least one solid, liquid or semi-liquid excipient or adjuvant.

4. Pharmaceutical preparation, **characterised by** a content of at least one compound of the formula I, according to Claim 1, and/or one of its physiologically acceptable salts.

5. Use of a compound of the formula I according to Claim 1 and/or one of its physiologically acceptable salts for the preparation of medicaments.

6. Use of a compound of the formula I according to Claim 1 and/or one of its physiologically acceptable salts for the preparation of a medicament for the treatment of diseases.

7. Use according to Claim 6 for the treatment of thromboses, cardiac infarction and/or tumours.

## Revendications

1. Composés de formule I

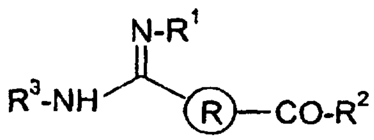

I,

dans laquelle

R désigne

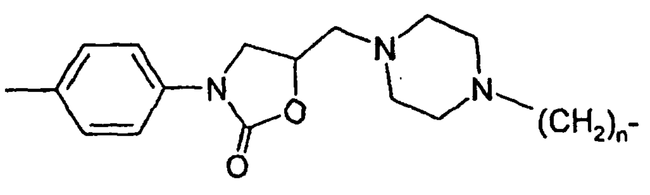

n désigne 1 ou 2,

$R^1$ désigne H,

$R^2$ désigne OA ou OAr,

$R^3$ désigne A-CO, Ar-CO, Hét-CO, Hét-O-CO, Ar-O-CO, A-O-CO, Ar-$SO_2$ ou A-$SO_2$,

A désigne alkyle ayant de 1 à 6 atomes de C,

Ar désigne aryle ayant de 6 à 10 atomes de C qui est non substitué ou mono-, di- ou trisubstitué par A, F, Cl, Br, I, OA, -O-$CH_2$-O-, COOA, COOH, $CF_3$, OH, $NO_2$, CN, $NH_2$, O-CO-A, NHA ou $NA_2$, diphénylméthyle ou benzyle et

Hét désigne un hétérocycle mono- ou bicyclique saturé, insaturé ou aromatique ayant de 1 à 4 atomes de N, de O et/ou de S, pouvant être non substitué ou monosubstitué par F, Cl, Br, $CF_3$, A, OH, OA, CN ou $NO_2$,

et les sels physiologiquement acceptables de ceux-ci.

**2.** (a) 3-p-(N-benzoylamidino)phényl-5-[4-(éthoxycarbonylméthyl)-pipérazinométhyl)oxazolidin-2-one,

(b) 3-p-(3-pyridylcarbonylamidino)phényl-5-[4-(éthoxycarbonylméthyl)pipérazinométhyl]oxazolidin-2-one,

(c) 3-p-(N-méthyl-4-pipéridyloxycarbonylamidino)phényl-5-[4-(éthoxycarbonylméthyl)pipérazinométhyl]oxazolidin-2-one,

(d) 3-p-[N-(éthoxycarbonylméthylcarbamoyl)amidino}phényl-5-[4-(tert-butoxycarbonyléthyl)pipérazinométhyl]oxazolidin-2-one,

(e) 3-p-(N-méthylsulfonylamidino)phényl-5-[4-(tert-butoxycarbonyléthyl)pipérazinométhyl]oxazolidin-2-one,

(f) 3-(4-(N-benzoylamidino)phényl]-5(R)-(4-éthoxycarbonylméthylpipérazinométhyl)oxazolidin-2-one,

(g) 3-(4-(N-benzoylamidino)phényl]-5(S)-(4-éthoxycarbonylméthylpipérazinométhyl)oxazolidin-2-one,

(h) 3-(4-(N-benzoylamidino)phényl]-5-(4-éthoxycarbonylméthylpipérazinométhyl)oxazolidin-2-one,

(l) 3-(4-(N-phénoxycarbonylamidino)phényl]-5-(4-éthoxycarbonylméthylpipérazinométhyl)oxazolidin-2-one,

(j) 3-(4-(N-1-naphtoylamidino)phényl]-5-(4-éthoxycarbonylméthylpipérazinométhyl)oxazolidin-2-one,

(k) 3-(4-(N-méthoxycarbonylamidino)phényl)-5-(4-éthoxycarbonylméthylpipérazinométhyl)oxazolidin-2-one,

(l) 3-(4-(N-benzoylamidino)phényl]-5-(4-éthoxycarbonyléthylpipérazinométhyl)oxazolidin-2-one,

(m) 3-(4-(N-benzoylamidino)phényl]-5(S)-(4-éthoxycarbonyléthylpipérazinométhyl)oxazolidin-2-one,

(n) 3-(4-(N-benzoylamidino)phényl]-5(R)-(4-éthoxycarbonyléthylpipérazinométhyl)oxazolidin-2-one,

(o) 3-(4-(N-benzoyloxycarbonylamidino)phényl]-(4-éthoxycarbonyléthylpipérazinométhyl)oxazolidin-2-one,

(p) 3-(4-(N-benzoyloxycarbonylamidino)phényl]-5(R)-(4-éthoxycarbonyléthylpipérazinométhyl)oxazolidin-2-one,

(q) 3-(4-(N-méthoxycarbonylamidino)phényl]-5-(4-éthoxycarbonyléthylpipérazinométhyl)oxazolidin-2-one,

(r) 3-(4-(N-benzoylamidino)phényl]-5-(4-tert-butoxycarbonyléthylpipérazinométhyl)oxazolidin-2-one,

(s) 3-(4-(N-benzoy!carbonylamidino)phényl]-5-(4-tert-butoxycarbonyléthylpipérazinométhyl)oxazolidin-2-one,

(t) 3-(4-(N-méthoxycarbonylamidino)phényl]-5-(4-tert-butoxycarbonyléthylpipérazinométhyl)oxazolidin-2-one,

(u) 3-(4-(N-méthoxybenzoylamidino)phényl]-5-(4-butoxycarbonylméthylpipérazinométhyl)oxazolidin-2-one,

(v) 3-(4-(N-méthylènedioxybenzoylamidino)phényl]-5-(4-éthoxycarbonylméthylpipérazinométhyl)oxazolidin-2-one,

(w) 3-(4-(N-méthoxybenzoylamidino)phényl]-5(R)-(4-éthoxycarbonylméthylpipérazinométhyl)oxazolidin-2-one,

(x) 3-(4-(N-3-trifluorométhylbenzoylamidino)phényl]-5(R)-(4-éthoxycarbonylméthylpipérazinométhyl)oxazolidin-2-one,

(y) 3-(4-(N-2-trifluorométhylbenzoylamidino)phényl]-5-(4-éthoxycarbonylméthylpipérazinométhyl)oxazolidin-2-one,

(z) 3-(4-(N-4-méthoxybenzoylamidino)phényl]-5-(4-éthoxycarbonyléthylpipérazinométhyl)oxazolidin-2-one,

(aa) 3-(4-(N-4-trifluorométhylbenzoylamidino)phényl]-5-(4-éthoxycarbonyléthylpipérazinométhyl)oxazolidin-2-one,

(bb) 3-(4-(N-3-trifluorométhylbenzoylamidino)phényl]-5-(4-éthoxycarbonyléthylpipérazinométhyl)oxazolidin-2-one,

(cc) 3-(4-(N-benzoylamidino)phényl]-5-(4-tert-butoxycarbonylméthylpipérazinométhyl)oxazolidin-2-one,

(dd) 3-(4-(N-éthoxycarbonylamidino)phényl]-5(R)-(4-éthoxycarbonylméthylpipérazinométhyl)oxazolidin-2-one,

(ee) 3-(4-(N-éthoxycarbonylamidino)phényl]-5(S)-(4-éthoxycarbonylméthylpipérazinométhyl)oxazolidin-2-one,

(ff) 3-(4-(N-isopropoxycarbonylamidino)phényl]-5(R)-(4-éthoxycarbonylméthylpipérazinométhyl)oxazolidin-2-one,

(gg) 3-(4-(N-méthoxycarbonylamidino)phényl]-5(R)-(4-éthoxycarbonylméthylpipérazinométhyl)oxazolidin-2-one,

(hh) 3-(4-(N-méthoxycarbonylamidino)phényl]-5-(4-tert-butoxycarbonylméthylpipérazinométhyl)oxazolidin-2-one,

selon la revendication 1, et les sels physiologiquement acceptables de ceux-ci.

**3.** Procédé de préparation d'une préparation pharmaceutique, **caractérisé en ce qu'**un composé de formule I selon la revendication 1 et/ou l'un de ses sels physiologiquement acceptables est mis sous une forme d'administration convenable conjointement avec au moins un excipient ou adjuvant solide, liquide ou semi-liquide.

**4.** Préparation pharmaceutique, **caractérisée par** une teneur en au moins un composé de formule 1 selon la revendication 1 et/ou l'un de ses sels physiologiquement acceptables.

**5.** Utilisation d'un composé de formule I selon la revendication 1 et/ou de l'un de ses sels physiologiquement acceptables pour la préparation de médicaments.

**6.** Utilisation d'un composé de formule I selon la revendication 1 et/ou de l'un de ses sels physiologiquement acceptables pour la préparation d'un médicament pour le traitement de maladies.

**7.** Utilisation selon la revendication 6 pour le traitement de thromboses, de l'infarctus du myocarde et/ou de tumeurs.